(19) Europäisches Patentamt
European Patent Office
Office européen des brevets

(11) Veröffentlichungsnummer: **0 039 519**
**B1**

(12) # EUROPÄISCHE PATENTSCHRIFT

(45) Veröffentlichungstag der Patentschrift: **02.05.84**

(51) Int. Cl.³: **C 07 D495/04**, A 61 K 31/55 // (C07D495/04, 333/00, 243/00)

(21) Anmeldenummer: **81103437.0**

(22) Anmeldetag: **06.05.81**

(54) **Substituierte Thienotricyclen, Verfahren zu ihrer Herstellung und sie enthaltende Arzneimittel.**

(30) Priorität: **07.05.80 CH 3581/80**
**02.02.81 CH 652/81**

(43) Veröffentlichungstag der Anmeldung: **11.11.81 Patentblatt 81/45**

(45) Bekanntmachung des Hinweises auf die Patenterteilung: **02.05.84 Patentblatt 84/18**

(84) Benannte Vertragsstaaten:
**AT BE CH DE FR GB IT LI LU NL SE**

(56) Entgegenhaltungen:
**DE-A- 1 795 176**
**DE-A- 1 931 487**
**DE-A- 2 552 403**
**DE-B- 1 795 183**
**FR-A- 1 505 795**
**US-A- 3 951 981**
**US-A- 3 953 430**
**US-A- 4 021 557**
**US-A- 4 115 574**
**US-A- 4 144 235**
**US-A- 4 168 269**
**US-A- 4 172 831**
**US-A- 4 263 207**

**Die Akte enthält technische Angaben, die nach dem Eingang der Anmeldung eingereicht wurden und die nicht in dieser Patentschrift enthalten sind.**

(73) Patentinhaber: **Byk Gulden Lomberg Chemische Fabrik GmbH**
**Byk-Gulden-Strasse 2**
**D-7750 Konstanz (DE)**

(72) Erfinder: **Figala, Volker, Dr.**
**Am Hochfirst 2**
**D-7753 Allensbach 4 (DE)**
Erfinder: **Riedel, Richard, Dr.**
**Salmannsweilergasse 36**
**D-7750 Konstanz (DE)**
Erfinder: **Rainer, Georg, Dr.**
**Josef-Anton-Feuchtmayer-Strasse 7**
**D-7750 Konstanz (DE)**
Erfinder: **Klemm, Kurt, Prof. Dr.**
**Im Weinberg 2**
**D-7753 Allensbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 039 519 B1

Jouve, 18, rue St-Denis, 75001 Paris, France

# Substituierte Thienotricyclen, Verfahren zu ihrer Herstellung, und sie enthaltende Arzneimittel

Die Erfindung betrifft substituierte Thienotricyclen, Verfahren zu ihrer Herstellung, ihre Verwendung und sie enthaltende Arzneimittel.

Die erfindungsgemäßen Verbindungen werden in der pharmazeutischen Industrie als Zwischenprodukte und zur Herstellung von Medikamenten verwendet.

In der deutschen Offenlegungsschrift DE-OS 1 795 176 wird bestimmten Dibenzodiazepinonen eine ulkushemmende und sekretionshemmende Wirkung zugeschrieben. Aus der US-Patentschrift US-PS 3, 953,430 sind substituierte Benzodiazepinone mit antidepressiver und analgetischer Wirkung bekannt. In der US-PS 4,168,269 werden substituierte Thienobenzodiazepinone mit analgetischer Wirkung beschrieben. Es wurden nun Thienobenzodiazepinone konzipiert, die neue interessante pharmakologische Wirkungen aufweisen.

Gegenstand der Erfindung sind substituierte Thienobenzodiazepinone der allgemeinen Formel I

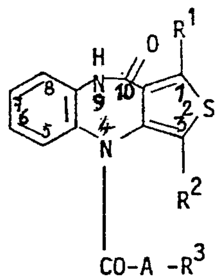

(I)

worin

$R^1$ ein Wasserstoffatom oder einen Alkylrest mit 1 bis 4 Kohlenstoffatomen bedeutet,

$R^2$ ein Halogenatom darstellt oder eine der Bedeutungen von $R^1$ hat,

$R^3$ ein Halogenatom oder die Gruppe $-N(R^4)R^5$ und

$R^4$ einen Alkylrest mit 1 bis 4 Kohlenstoffatomen oder einen Alkenylrest mit 3 bis 5 Kohlenstoffatomen bedeuten,

$R^5$ eine der Bedeutungen von $R^4$ hat oder die Gruppe $-(CH_2)_m-N(R^6)R^7$ darstellt oder

$R^4$ und $R^5$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl-, Ethyl- oder Benzylgruppe substituierte Piperazin-1-yl-gruppe, eine 2,4-Dimethylpiperazin-1-yl-gruppe oder eine in 4-Position durch eine Methyl- oder Ethylgruppe substituierte Hexahydro-1H-1,4-diazepin-1-yl-gruppe bedeuten und

$R^6$ eine Alkylgruppe mit 1 bis 4 Kohlenstoffatomen,

$R^7$ eine Alkylgruppe mit 1 bis 4 Kohlenstoffatomen,

A eine geradkettige oder verzweigte Alkylengruppe mit 1 bis 5 Kohlenstoffatomen und

m 2 oder 3 bedeuten,

sowie ihre Säureadditionssalze.

Alkylreste mit 1 bis 4 Kohlenstoffatomen sind der Methyl-, Ethyl-, Propyl-, Isopropyl-, n-Butyl, Isobutyl-, sek.-Butyl-, tert.-Butylrest. Von den Alkylresten sind bei $R^1$, $R^2$, $R^4$, $R^5$, $R^6$ und $R^7$ der Methyl- und Ethylrest bevorzugt. Besonders bevorzugt als Alkylrest $R^1$ und $R^2$ ist der Methylrest.

Als Alkenylreste mit 3 bis 5 Kohlenstoffatomen seien der Allylrest und der 2-Methylallylrest genannt.

Halogenatome $R^2$ sind das Bromatom, insbesondere das Chloratom.

Halogenatome $R^3$ (Hal) sind das Iod-, das Brom- und insbesondere das Chloratom.

Alkylengruppen mit 1 bis 5 Kohlenstoffatomen sind die Trimethylen-, Tetramethylen-, Pentamethylen-, Propylen-, Ethylmethylengruppe, bevorzugt die Ethylengruppe, insbesondere die Methylengruppe.

Als Salze kommen beliebige Säureadditionssalze in Betracht. Besonders erwähnt seien die pharmakologisch verträglichen Salze der in der Galenik üblicherweise verwendeten anorganischen und organischen Säuren. Pharmakologisch unverträgliche Salze werden durch dem Fachmann bekannte Verfahren in pharmakologisch verträgliche Salze übergeführt. Als solche seien beispielsweise genannt wasserlösliche oder wasserunlösliche Säureadditionssalze, wie das Hydrobromid, Hydroiodid, Nitrat, Acetat, Benzoat, Hibenzat [2-(4-Hydroxybenzoyl)-benzoat], Fendizoat (2-[(2'-Hydroxy-4-biphenylyl)-carbonyl]-benzoat), Propionat, Butyrat, Sulfosalicylat, Laurat, Oxalat, Amsonat (4,4'-Diaminostilben-2,2'-disulfonat), Embonat [4,4'-Methylen-bis-(3-hydroxy-2-naphthoat)], Metembonat [4,4'-Methylen-bis-(3-methoxy-2-naphthoat)], Stearat, 2-Hydroxy-3-naphthoat, 3-Hydroxy-2-naphthoat, insbesondere das Hydrochlorid, Phosphat, Sulfat, Citrat, Gluconat, Maleat, Malat, Fumarat, Succinat, Tartrat, Tosilat (p-Toluolsulfonat), Mesilat (Methansulfonat), Amidosulfonat.

Eine Ausgestaltung der Erfindung sind substituierte Thienobenzodiazepinone der allgemeinen Formel I*

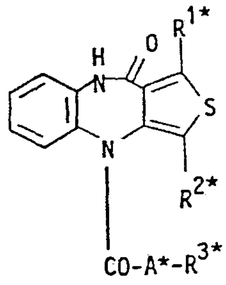

(I*)

worin

$R^{1*}$ ein Wasserstoffatom, einen Methyl- oder Ethylrest bedeutet,

$R^{2*}$ ein Chloratom darstellt oder eine der Bedeutungen von $R^{1*}$ hat,

$R^{3*}$ ein Chloratom und

A* eine geradkettige oder verzweigte Alkylengruppe mit 1 oder 2 Kohlenstoffatomen bedeuten.

Bevorzugte Vertreter der Ausgestaltung I* sind solche, in denen $R^{1*}$ ein Wasserstoffatom oder einen Methylrest, $R^{2*}$ ein Wasserstoffatom oder einen Methylrest und A* eine Methylengruppe bedeuten.

Besonders bevorzugte Vertreter der Ausgestaltung I* sind solche, in denen $R^{1*}$ und $R^{2*}$ ein Wasserstoffatom, $R^{3*}$ ein Chloratom und A* eine Methylengruppe bedeuten.

Eine weitere Ausgestaltung der Erfindung sind substituierte Thienobenzodiazepinone der allgemeinen Formel I**

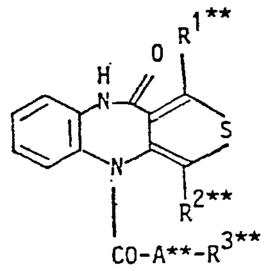

(I**)

worin

$R^{1**}$ ein Wasserstoffatom, einen Methyl- oder Ethylrest bedeutet,

$R^{2**}$ ein Chloratom darstellt oder eine der Bedeutungen von $R^{1**}$ hat,

$R^{3**}$ die Gruppe $—N(R^{4**})R^{5**}$ und

$R^{4**}$ einen Alkylrest mit 1 bis 4 Kohlenstoffatomen oder einen Alkenylrest mit 3 bis 4 Kohlenstoffatomen bedeuten,

$R^{5**}$ die Bedeutung von $R^{4**}$ hat oder die Gruppe $—(CH_2)_{m**}—N(R^{6**})R^{7**}$ darstellt oder

$R^{4**}$ und $R^{5**}$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine ggf. in 4-Position durch eine Methyl-, Ethyl- oder Benzylgruppe substituierte Piperazin-1-yl-gruppe, eine 2,4-Dimethyl-piperazin-1-yl-gruppe oder eine in 4-Position durch eine Methyl- oder Ethylgruppe substituierte Hexahydro-1H-1,4-diazepin-1-yl-gruppe bedeuten und

$R^{6**}$ eine Methyl- oder Ethylgruppe,

$R^{7**}$ eine Methyl- oder Ethylgruppe,

m** 2 oder 3,

A** eine geradkettige oder verzweigte Alkylengruppe mit 1 oder 2 Kohlenstoffatomen bedeuten, und ihre Säureadditionssalze.

Eine Gruppe von Vertretern der Ausgestaltung I** sind solche, in denen $R^{1**}$ ein Wasserstoffatom, einen Methyl- oder Ethylrest bedeutet, $R^{2**}$ ein Chloratom darstellt oder eine der Bedeutungen von $R^{1**}$ hat; $R^{4**}$ einen Methyl- oder Ethylrest bedeutet, $R^{5**}$ die Bedeutung von $R^{4**}$ hat oder die Gruppe $(CH_2)_{m**}—N(R^{6**})R^{7**}$ darstellt oder $R^{4**}$ und $R^{5**}$ gemeinsam unter Einschluß des Stickstoffatoms einen Pyrrolidino-, Piperidino- oder Hexahydroazepin-1-yl-rest, $R^{6**}$ und $R^{7**}$ einen Methyl- oder Ethylrest, m** 2 und A** eine Methylengruppe bedeuten, sowie ihre pharmakologisch verträglichen Säureadditionssalze.

Eine andere Gruppe von Vertretem der Ausgestaltung I** sind solche, in denen $R^{1**}$ ein Wasserstoffatom, einen Methyl- oder Ethylrest bedeutet, $R^{2**}$ ein Chloratom darstellt oder eine der Bedeutungen von $R^{1**}$ hat; $R^{4**}$ und $R^{5**}$ gemeinsam unter Einschluß des Stickstoffatoms eine in 4-Stellung durch eine Methyl-, Ethyl- oder Benzylgruppe substituierte Piperazin-1-yl-gruppe, eine 2,4-Dimethyl-piperazin-1-yl-gruppe oder eine in 4-Stellung durch eine Methyl- oder Ethylgruppe substituierte hexahydro-1H-1,4-diazepin-1-yl-gruppe und A** eine Methylengruppe bedeuten, sowie ihre pharmakologisch verträglichen Säureadditionssalze.

Bevorzugte Vertreter der Ausgestaltung I** sind solche in denen $R^{1**}$ ein Wasserstoffatom oder einen Methylrest, $R^{2**}$ ein Wasserstoffatom oder einen Methylrest, $R^{4**}$ und $R^{5**}$ gemeinsam unter Einschluß des Stickstoffatoms eine in 4-Stellung durch eine Methylgruppe substituierte Piperazin-1-yl-gruppe und A** eine Methylengruppe bedeuten, sowie ihre pharmakologisch verträglichen Säureadditionssalze.

Als Vertreter der erfindungsgemäßen Verbindungen seien beispielsweise genannt :

9,10-Dihydro-4-[2-(di-n-propylamino)-propionyl]-4H-thieno [3,4-b] [1,5] benzodiazepin-10-on,
4-[4-(Di-n-butylamino)-butyryl]-9,10-dihydro-4H-thieno [3,4-b] [1,5] benzodiazepin-10-on,
4-[2-(Diethylamino)-propionyl]-9,10-dihydro-4H-thieno [3,4-b] [1,5] benzodiazepin-10-on,
4-[5-(Diisopropylamino)-valeryl]-9,10-dihydro-4H-thieno [3,4-b] [1,5] benzodiazepin-10-on,
4-[Diisobutylaminoacetyl]-9,10-dihydro-3-methyl-4H-thieno [3,4-b] [1,5] benzodiazepin-10-on,
4-[N-n-Butyl-tert.-butylaminoacetyl]-9,10-dihydro-3-methyl-4H-thieno [3,4-b] [1,5] benzodiazepin-10-on,
4-[4-(Diallylamino)-butyryl]-9,10-dihydro-1,3-dimethyl-4H-thieno [3,4-b] [1,5] benzodiazepin-10-on,
4-[Di-sek.-butylaminoacetyl]-9,10-dihydro-4H-thieno [3,4-b] [1,5] benzodiazepin-10-on,
4-[2-(N-Ethyl-n-butylamino)-propionyl]-9,10-dihydro-4H-thieno [3,4-b] [1,5] benzodiazepin-10-on,
9,10-Dihydro-3-methyl-4-[N-methyl-sek.-butylaminoacetyl]-4H-thieno [3,4-b] [1,5] benzodiazepin-10-on,
9,10-Dihydro-4-[5-(N-methyl-tert.-butylamino)-valeryl]-4H-thieno [3,4-b] [1,5] benzodiazepin-10-on,
9,10-Dihydro-4-[2-piperidinopropionyl]-4H-thieno [3,4-b] [1,5] benzodiazepin-10-on,
4-[4-(Hexahydroazepin-1-yl)-butyryl]-9,10-dihydro-4H-thieno [3,4-b] [1,5] benzodiazepin-10-on,
4-[3-(Di-n-butylamino)-propionyl]-9,10-dihydro-4H-thieno [3,4-b] [1,5] benzodiazepin-10-on,
4-[3-(Diallylamino)-propionyl]-9,10-dihydro-4H-thieno [3,4-b] [1,5] benzodiazepin-10-on,
4-[3-(Di-sek.-butylamino)-propionyl]-9,10-dihydro-4H-thieno [3,4-b] [1,5]-benzodiazepin-10-on,
4-[3-(N-n-Butyl-tert.-butylamino)-propionyl]-9,10-dihydro-4H-thieno [3,4-b] [1,5] benzodiazepin-10-on,
4-[3-(N-Ethyl-n-butylamino)-propionyl]-9,10-dihydro-4H-thieno [3,4-b] [1,5] benzodiazepin-10-on,
9,10-Dihydro-4-[3-(N-methyl-sek.-butylamino)-propionyl]-4H-thieno [3,4-b] [1,5] benzodiazepin-10-on,
9,10-Dihydro-4-[3-piperidinopropionyl]-4H-thieno [3,4-b] [1,5] benzodiazepin-10-on,
9,10-Dihydro-1-methyl-4-[(4-methylpiperazin-1-yl)-acetyl]-4H-thieno [3,4-b] [1,5] benzodiazepin-10-on,
4-[3-(hexahydroazepin-1-yl) propionyl]-9,10-dihydro-4H-thieno [3,4-b] [1,5] benzodiazepin-10-on,
bevorzugt
9,10-Dihydro-4-[(4-methylpiperazin-1-yl)-acetyl]-4H-thieno [3,4-b] [1,5]-benzodiazepin-10-on,
9,10-Dihydro-1,3-dimethyl-4-[(4-methylpiperazin-1-yl)-acetyl]-4H-thieno-[3,4-b] [1,5] benzodiazepin-10-on und
9,10-Dihydro-3-methyl-4-[4-methylpiperazin-1-yl)-acetyl]-4H-thieno [3,4-b] [1,5] benzodiazepin-10-on sowie ihre pharmakologisch verträglichen Säureadditionssalze.

Die substituierten Thienobenzodiazepinone der allgemeinen Formel I und ihre Säureadditionssalze bzw. die Ausgestaltungen I* und I** besitzen wertvolle Eigenschaften, die sie gewerblich verwertbar machen. Die substituierten Thienobenzodiazepinone der allgemeinen Formel I, in der $R^3$ die Gruppe $N(R^4)R^5$ bedeutet und $R^4$ und $R^5$ die oben angegebenen Bedeutungen haben, bzw. die der Ausgestaltung I** sind durch eine ausgezeichnete Magen- und Darmschutzwirkung bei Warmblütlern gekennzeichnet, sie Hemmen z. b. die Bildung von Magengeschwüren. Ferner weisen sie, bedingt durch eine geringe Toxizität und das Fehlen wesentlicher Nebenwirkungen eine günstige therapeutische Breite auf. Die substituierten Thienobenzodiazepinone der allgemeinen Formel I, in der $R^3$ ein Halogenatom (Hal) bedeutet und Hal die oben angegebene Bedeutung hat, bzw. die der Ausgestaltung I* sind wertvolle Zwischenprodukte bei der Herstellung der pharmakologisch wirksamen und therapeutisch einsetzbaren erfindungsgemäßen Verbindungen.

Die ausgezeichnete Wirksamkeit der pharmakologisch wirksamen substituierten Thienobenzodiazepinone und ihrer pharmakologisch, d. h. biologisch verträglichen Säureadditionssalze ermöglicht ihren Einsatz in der Human- und auch in der Veterinärmedizin, wobei sie zur Behandlung und Prophylaxe von Krankheiten, die auf Erkrankungen des Magens oder Darms beruhen, verwendet werden. Beispielsweise werden akuter und chronischer Ulcus ventriculi und Ulcus duodeni, Gastritis oder hyperacider Reizmagen bei Mensch oder Tier behandelt.

Die erfindungsgemäßen Verdindungen werden daher bei der Behandlung von Säugetieren, die an einer der oben genannten Krankheiten erkrankt sind, angewendet, wobei man dem erkrankten Säugetier eine therapeutisch wirksame und pharmakologisch verträgliche Menge einer oder mehrerer Verbindungen der allgemeinen Formeln I, I**, deren bevorzugter Vertreter und/oder deren Salze verabreicht. Gegenstand der Erfindung sind somit auch die erfindungsgemäßen Verbindungen bei der Anwendung zur Bekämpfung der oben angegebenen Krankheiten. Ebenso umfaßt die Erfindung die Verwendung der erfindungsgemäßen Verbindungen bei der Herstellung von Arzneimitteln, die zur Bekämpfung der angeführten Krankheiten eingesetzt werden.

Ein weiterer Gegenstand der Erfindung sind Arzneimittel, die ein oder mehrere Thienobenzodiazepinone der allgemeinen Formel Ia

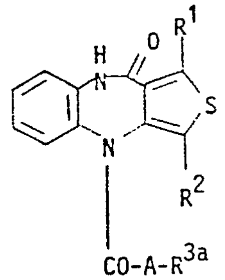

(Ia)

worin

$R^1$ ein Wasserstoffatom oder einen Alkylrest mit 1 bis 4 Kohlenstoffatomen bedeutet,

$R^2$ ein Halogenatom darstellt oder eine der Bedeutungen von $R^1$ hat,

$R^{3a}$ die Gruppe $-N(R^4)R^5$ und

$R^4$ einen Alkylrest mit 1 bis 4 Kohlenstoffatomen oder einen Alkenylrest mit 3 bis 5 Kohlenstoffatomen bedeuten,

$R^5$ eine der Bedeutungen von $R^4$ hat oder die Gruppe $-(CH_2)_m-N(R^6)R^7$ darstellt oder

$R^4$ und $R^5$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl-, Ethyl- oder Benzylgruppe substituierte Piperazin-1-yl-gruppe, eine 2,4-Dimethyl-piperazin-1-yl-gruppe oder eine in 4-Position durch eine Methyl- oder Ethylgruppe substituierte Hexahydro-1H-1,4-diazepin-1-yl-gruppe bedeuten und

$R^6$ eine Alkylgruppe mit 1 bis 4 Kohlenstoffatomen,

$R^7$ eine Alkylgruppe mit 1 bis 4 Kohlenstoffatomen,

A eine geradkettige oder verzweigte Alkylengruppe mit 1 bis 5 Kohlenstoffatomen und

m 2 oder 3 bedeuten,

und/oder ihre pharmakologisch verträglichen Säureadditionssalze enthalten.

Ausgestaltungen der Arzneimittel sind solche, die Thienobenzodiazepinone der Formel I** oder ihre bevorzugten Vertreter und/oder ihre pharmakologisch verträglichen Säureadditionssalze enthalten.

Die Arzneimittel werden nach an sich bekannten Verfahren hergestellt. Als Arzneimittel werden die erfindungsgemäßen Verbindungen entweder als solche oder vorzugsweise in Kombination mit geeigneten pharmazeutischen Trägerstoffen eingesetzt. Enthalten die neuen pharmazeutischen Zubereitungen neben den erfindungsgemäßen Verbindungen pharmazeutische Trägerstoffe, beträgt der Wirkstoffgehalt dieser Mischungen 0,5 bis 95, vorzugsweise 15 bis 75, Gewichtsprozent der Gesamtmischung. Die Arzneimittel werden beispielsweise für die orale, rektale oder parenterale (intravenöse, intramusculäre, subcutane) Gabe in geeigneten Dosen formuliert, zum Beispiel als Tablette, Dragee, Kapsel, Suppositorium oder gemessene Volumenmenge eines Pulvers eines Granulates, einer Lösung, einer Emulsion oder einer Suspension.

Die Tagesdosis bei oraler Gabe liegt im allgemeinen für Säugetiere zwischen 0,01 und 5, vorzugsweise 0,05 und 2,5, insbesondere 0,1 und 1,5 mg/kg Körpergewicht, die gegebenenfalls in Form mehrerer, vorzugsweise 1 bis 3 Einzelgaben zur Erzielung der gewünschten Ergebnisse verabreicht wird.

Die pharmazeutischen Zubereitungen bestehen bevorzugt aus den erfindungsgemäßen Wirkstoffen und nichttoxischen, pharmazeutisch verträglichen Arzneimittelträgern, die als Zumischung oder Verdünnungsmittel in fester, halbfester oder flüssiger Form oder als Umhüllungsmittel, beispielsweise in Form einer Kapsel, eines Tablettenüberzugs, eines Beutels oder eines anderen Behältnisses, für den therapeutisch aktiven Bestandteil in Anwendung kommen. Ein Trägerstoff kann z. B. als Vermittler für die Arzneimittelaufnahme durch den Körper, als Formulierungshilfsmittel, als Süßungsmittel, als Geschmackskorrigenz, als Farbstoff oder als Konservierungsmittel dienen.

Sollen die erfindungsgemäßen substituierten Thienobenzodiazepinone und/oder ihre pharmakologisch verträglichen Säureadditionssalze zur Behandlung der angegebenen Krankheiten eingesetzt werden, so können die pharmazeutischen Zubereitungen auch einen oder mehrere pharmakologisch aktive Bestandteile anderer Arzneimittelgruppen, wie Antacida, beispielsweise Aluminiumhydroxid, Magnesiumaluminat ; Sekretionshemmer, wie H2-Blocker, z. B. Cimetidin ; Magen- und Darmtherapeutika, z. B. Metoclopramid, Bromoprid, Tiaprid ; Tranquilizer, wie Benzodiazepine, beispielsweise Diazepam ; Spasmolytika, z. B. Bietamiverin, Camylofin ; Anticholinergica, z. B. Oxyphencyclimin, Phencarbamid ; Glucocorticoide, wie Prednisolon, Fluocortolon, Betamethason ; nichtsteroidale Antiphlogistika, wie Arylessigsäuren und -propionsäuren, Heteroarylessigsäuren und -propionsäuren, Benzothiazincarboxamiddioxide, Pyrazolidindione, Chinazolinone, z. B. Ibuprofen, Naproxen, Diclofenac, Fenbufen, Indometacin, Lonazolac, Sudoxicam, Piroxicam, Phenylbutazon, Bumadizon-Calcium, Proquazon ; Lokalanaesthetika, beispielsweise Tetracain, Procain ; gegebenenfalls auch Fermente, Vitamine, Aminosäuren etc. enthalten.

Ein weiterer Gegenstand der Erfindung ist ein Verfahren zur Herstellung der substituierten Thienobenzodiazepinone der allgemeinen Formel I

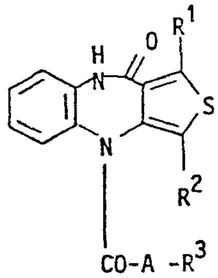

(I)

worin

$R^1$ ein Wasserstoffatom oder einen Alkylrest mit 1 bis 4 Kohlenstoffatomen bedeutet,

$R^2$ ein Halogenatom darstellt oder eine der Bedeutungen von $R^1$ hat,

$R^3$ ein Halogenatom oder die Gruppe $—N(R^4)R^5$ und

$R^4$ einen Alkylrest mit 1 bis 4 Kohlenstoffatomen oder einen Alkenylrest mit 3 bis 5 Kohlenstoffatomen bedeuten,

$R^5$ eine der Bedeutungen von $R^4$ hat oder die Gruppe $—(CH_2)_m—N(R^6)R^7$ darstellt oder

$R^4$ und $R^5$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl-, Ethyl- oder Benzylgruppe substituierte Piperazin-1-yl-gruppe, eine 2,4-Dimethylpiperazin-1-yl-gruppe oder eine in 4-Position durch eine Methyl- oder Ethylgruppe substituierte Hexahydro-1H-1,4-diazepin-1-yl-gruppe bedeuten und

$R^6$ eine Alkylgruppe mit 1 bis 4 Kohlenstoffatomen,

$R^7$ eine Alkylgruppe mit 1 bis 4 Kohlenstoffatomen,

A eine geradkettige oder verzweigte Alkylengruppe mit 1 bis 5 Kohlenstoffatomen und

m 2 oder 3 bedeuten,

sowie ihrer Säureadditionssalze.

Das Verfahren ist dadurch gekennzeichnet, daß man Thienobenzodiazepinone der allgemeinen Formel II

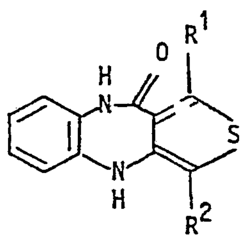

(II)

worin

$R^1$ und $R^2$ die oben angegebene Bedeutung haben, acyliert und gegebenenfalls anschließend aminiert und/oder erhaltene Basen in die Säureadditionssalze oder erhaltene Säureadditionssalze in die freie Base oder pharmakologisch verträgliche Säureadditionssalze überführt.

Die Acylierung und die sich gegebenenfalls anschließende Aminierung erfolgt nach an sich bekannten Methoden.

Zur Herstellung der Thienobenzodiazepinone der allgemeinen Formel I, in der $R^3$ —Hal bedeutet, werden die Ausgangsverbindung der Formel II, worin $R^1$ und $R^2$ die oben angegebenen Bedeutungen haben, oder ihre Säureadditionssalze mit Verbindungen der allgemeinen Formeln III Hal—A—CO—Hal' (III) oder IV $[Hal—A—CO]_2O$ (IV), worin Hal' eine der Bedeutungen von Hal hat und A und Hal die oben angegebenen Bedeutungen haben, umgesetzt. Diese Acylierung wird ohne oder vorzugsweise in einem inerten Lösungsmittel bei Raumtemperatur oder erhöhter Temperatur, maximal bei der Siedetemperatur des Lösungsmittels, gegebenenfalls in Gegenwart einer Hilfsbase und/oder eines Acylierungskatalysators, vorgenommen. Die Säurehalogenide III sind gegenüber den Säureanhydriden IV bevorzugt. Als Säurehalogenid III ist Chloracetylchlorid, als Säureanhydrid IV ist Chloressigsäureanhydrid bevorzugt. Als Lösungsmittel seien beispielsweise genannt aromatische Kohlenwasserstoffe, wie Toluol, Xylol oder Chlorbenzol; offenkettige oder cyclische Ether, wie Diisopropylether oder Dioxan; chlorierte Kohlenwasserstoffe, wie Dichlorethan, andere Lösungsmittel, wie Pyridin, Acetonitril oder Dimethylformamid.

Als Hilfsbasen seien z. B. tertiäre organische Basen, wie Triethylamin und Ethyldiisopropylamin, oder Pyridin; oder anorganische Basen, wie wasserfreie Alkalimetall- oder Erdalkalimetallcarbonate oder

-hydrogencarbonate oder Erdalkalimetalloxide, genannt. Als Acylierungskatalysatoren kommen beispielsweise in Frage Imidazol, Pyridin oder 4-Dimethylaminopyridin.

Das Verfahren zur Herstellung der Zwischenprodukte der allgemeinen Formel I ist also dadurch gekennzeichnet, daß man ein Thienobenzodiazepinon der allgemeinen Formel II mit Verbindungen der allgemeinen Formeln III oder IV acyliert. Bei der Herstellung der Zwischenprodukte der allgemeinen Formel I* werden entsprechende Ausgangsstoffe eingesetzt.

Zur Herstellung der substituierten Thienobenzodiazepinone der allgemeinen Formel I, in der $R^1$, $R^2$ und A die oben angegebene Bedeutung haben und $R^3$ eine Gruppe $—N(R^4)R^5$ bedeutet, wird das erhaltene Reaktionsprodukt der Formel I, worin $R^3$ —Hal bedeutet, mit sekundären Aminen der allgemeinen Formel V $HN(R^4)R^5$ (V) umgesetzt, wobei $R^4$, $R^5$ und Hal die obige Bedeutung haben.

Die Aminierung erfolgt in einem inerten Lösungsmittel bei Temperaturen zwischen 0° und Siedetemperatur des Lösungsmittels entweder mit wenigstens 2 Molen sekundärem Amin V oder mit 1 bis 2 Molen sekundärem Amin V und einer Hilfsbase. Als Lösungsmittel kommen beispielsweise in Frage chlorierte Kohlenwasserstoffe, wie Methylenchlorid, Chloroform oder Dichlorethan ; offenkettige oder cyclische Ether, wie Diethylether, Tetrahydrofuran oder Dioxan ; aromatische Kohlenwasserstoffe, wie Benzol, Toluol, Xylol, Chlorbenzol oder Pyridin ; Alkohole, wie Ethanol oder Isopropanol ; Ketone, wie Aceton ; Acetonitril oder Dimethylformamid. Als Hilfsbase seien beispielsweise genannt tertiäre organische Basen, wie Triethylamin, N-Methylpiperidin, Diethylanilin oder Pyridin, oder anorganische Basen, wie Alkalimetall- oder Erdalkalimetallcarbonate oder -hydrogencarbonate, Erdalkalimetallhydroxide oder -oxide. Gegebenenfalls kann die Reaktion durch Zusatz von Alkalimetalliodiden beschleunigt werden. Die Reaktionszeiten betragen je nach Menge und Art des eingesetzten Amins V zwischen 15 Minuten und 80 Stunden. Bei Umsetzung von Ausgangsverbindungen, in denen A eine Alkylengruppe mit 2 bis 5 Kohlenstoffatomen darstellt, kann die Reaktion auch unter Abspaltung von H—Hal verlaufen ; die intermediär gebildete, gegebenenfalls isolierbare Alkenylverbindung reagiert mit dem sekundären Amin V zum gleichen Endprodukt.

Zur Herstellung der substituierten Thienobenzodiazepinone der allgemeinen Formel I, in der $R^1$, $R^2$ und A die oben angegebene Bedeutung haben und $R^3$ eine Gruppe $—N(R^4)R^5$ bedeutet, werden alternativ Thienobenzodiazepinone der allgemeinen Formel II, worin $R^1$ und $R^2$ die oben angegebene Bedeutung haben, mit Verbindungen der allgemeinen Formel IX

$$Z—CO—A—N(R^4)R^5 \qquad (IX)$$

worin A, $R^4$ und $R^5$ die oben angegebenen Bedeutungen haben und Z eine Fluchtgruppe (= Abgangsgruppe) darstellt, acyliert.

Die Umsetzung der Verbindungen II mit den Säurederivaten IX erfolgt in an sich bekannter Weise. Die Fluchtgruppe Z ist eine Gruppe, die gemeinsam mit der Carbonylgruppe, an die sie gebunden ist, ein reaktives Carbonsäurederivat bildet. Als reaktive Carbonsäurederivate seien beipielsweise Säurehalogenide, -ester, -anhydride oder gemischte -anhydride, wie sie aus Salzen der entsprechenden Säure (Z = OH) und Säurechloriden, wie Phosphoroxidchlorid oder Chlorameisensäureester, gebildet werden, genannt. Bevorzugt wird die Reaktion mit den gemischten Anhydriden IX starker Mineralsäuren, insbesondere der Chlorphosphorsäure, durchgeführt. Die Reaktion wird gegebenenfalls in Gegenwart eines säurebindenden Mittels (Protonenakzeptors) durchgeführt. Als geeignete Protonenakzeptoren seien beispielsweise genannt Alkalimetallcarbonate oder -hydrogencarbonate, wie Natriumcarbonat oder Kaliumhydrogencarbonat ; tertiäre organische Amine, wie Pyridin, Triethylamin, Ethyldiisopropylamin ; oder Natriumhydrid. Die Reaktion wird bei Temperaturen zwischen − 25° und 50° in einem inerten Lösungsmittel, vorzugsweise in Dimethylformamid, durchgeführt.

Zur Herstellung der substituierten Thienobenzodiazepinone der allgemeinen Formel I, in der $R^1$, $R^2$ und A die oben angegebene Bedeutung haben und $R^3$ eine Gruppe $—N(R^4)R^5$ bedeutet, in der $R^4$ und $R^5$ gemeinsam unter Einschluß des Stickstoffatoms, an das sie gebunden sind, eine in 4-Position durch eine Methyl- oder Ethylgruppe substituierte Piperazin-1-yl-gruppe oder eine in 4-Position durch eine Methyl- oder Ethylgruppe substituierte Hexahydro-1H-1,4-diazepin-1-yl-gruppe bedeuten, werden alternativ erhaltene Piperazinylthienobenzodiazepinone der allgemeinen Formel X

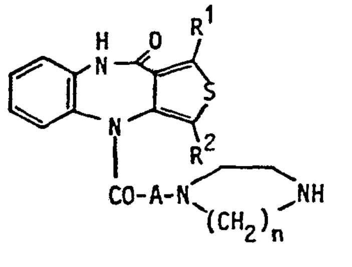

(X)

worin $R^1$, $R^2$ und A die oben angegebene Bedeutung haben und n 2 oder 3 bedeuten, methyliert oder ethyliert.

Die Methylierung und die Ethylierung werden nach an sich bekannten Verfahren durchgeführt. So kommen beispielsweise als Methylierungs- und Ethylierungsmittel für Verbindungen der allgemeinen Formel X in Betracht: Methyl- und Ethylester starker Säuren, beispielsweise von Schwefelsäure, Phosphorsäure oder p-Toluolsulfonsäure, oder Methyl- oder Ethylhalogenide, die zwischen 0 °C und 50 °C gegebenenfalls in Gegenwart eines Protonenakzeptors in einem wässerigen oder nichtwässerigen Medium eingesetzt werden, wie es z. B. in Houben-Weyl Band XI/1, Seite 24 ff. und S. 205 ff., Georg-Thieme-Verlag, Stuttgart (1957) beschrieben ist; Mischungen von Formaldehyd oder Acetaldehyd mit einem Reduktionsmittel (Methode der reduktiven Alkylierung), wobei als Reduktionsmittel naszierender Wasserstoff (z. B. aus Zink und Salzsäure), Wasserstoff in Gegenwart eines Hydrierungskatalysators, wie Platin oder Raney-Nickel, Ameisensäure oder komplexe Metallhydride, wie Natriumborhydrid oder Natriumcyanborhydrid, in Frage kommen. Methoden der reduktiven Alkylierung sind z. B. ausführlich beschrieben in Houben-Weyl, band XI/1, Seite 602 ff., Georg-Thieme-Verlag, Stuttgart (1957); W.S. Emerson, Organic Reactions, Band 4, S. 174 ff., John Wiley and Sons, New York (1948); M.L. Moore, idid, Band 5, S. 301 ff. (1949); C.A. Buehler, D.E. Pearson, Survey of Organic Synthesis, Band 1, S. 424-429 (1970), Band 2, 403-407 (1977), John Wiley and Sons, New York; S.R. Sandler, W. Karo, Organic Functional Group Preparations, Band 1, S. 345 ff. (1968), Academic Press, New York. Bevorzugt wird die Methylierung als reduktive Methylierung vorgenommen.

Das Verfahren zur Herstellung der pharmakologisch wirksamen Thienobenzodiazepinone der allgemeinen Formel I ist also dadurch gekennzeichnet, daß man Verbindungen der Formel I, worin $R^3$ —Hal bedeutet, mit Verbindungen der allgemeinen Formel V umsetzt oder daß man Thienobenzodiazepinone der allgemeinen Formel II mit Säurederivaten IX acyliert oder daß man Verdingungen X methyliert oder ethyliert und jeweils gegebenenfalls anschließend die erhaltene Base in ein pharmakologisch verträgliches Säureadditionssalz oder ein erhaltenes Säureadditionssalz in die freie Base oder ein pharmakologisch verträgliches Säureadditonssalz überführt.

Säureadditionssalze erhält man durch Auflösen der erhaltenen freien Base in einem geeigneten Lösungsmittel, z. B. Wasser, Aceton, einem Alkanol, wie Ethanol oder Isopropanol, einem offenkettigen oder cyclischen Ether, wie Diethylether oder Tetrahydrofuran, das die gewünschte Säure enthält oder dem die gewünschte Säure anschließend zugegeben wird. Die Salze werden durch Filtrieren, Ausfällen mit einem Nichtlösungsmittel für das Säureadditionssalz oder durch Verdampfen des Lösungsmittels gewonnen. Salze können auch durch Überführung in die Base und weitere Umsetzung mit einer anderen Säure in andere Salze, z. B. pharmakologisch verträgliche Säureadditionssalze, übergeführt werden.

Erhaltene Salze können z. B. durch Alkalisieren mit wäßrigem Natrium- oder Kaliumhydroxid in die freie Base umgewandelt werden, die dann durch gegeeignete Maßnahmen, z. B. Lösungsmittelextraktion mit einem nicht mit Wasser mischbaren Lösungsmittel, wie Chloroform, Diethylether, Toluol, gewonnen wird.

Die Herstellung der Ausgangsverbindungen der allgemeinen Formel II erfolgt gemäß oder analog der US-PS 3,953,430 nach folgendem Reaktionsschema:

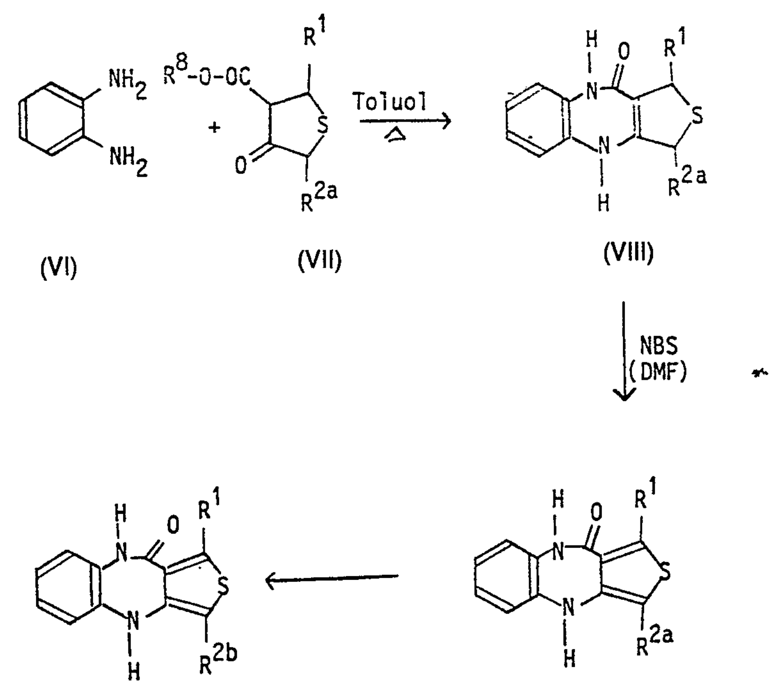


(VI) (VII) (VIII)

(IIb) (IIa)

Phenylendiamin (VI) wird mit den Tetrahydrothiophencarbonsäurederivaten VII, in denen $R^1$ die oben genannte Bedeutung hat sowie $R^{2a}$ ein Wasserstoffatom oder eine Alkylgruppe mit 1 bis 4 Kohlenstoffatomen und $R^8$ ein Wasserstoffatom oder eine Alkylgruppe mit 1 bis 5 Kohlenstoffatomen bedeuten, in inerten Lösungsmitteln, z. B. Toluol, unter Erhitzen zu den Tetrahydrothienobenzodiazepinonen VIII umgesetzt. Die Verbindungen VIII werden mit einem geeigneten Dehydrierungsmittel, z. B. N-Bromsuccinimid in Dimethylformamid, zu den Dihydrothienobenzodiazepinonen IIa dehydriert. Durch Chlorierung oder Bromierung mit geeigneten Halogenierungsmitteln werden die Vertreter IIa, in denen $R^{2a}$ ein Wasserstoffatom bedeutet, zu den Halogenderivaten IIb, in denen $R^{2b}$ ein Chlor- oder Bromatom bedeutet, umgesetzt.

Die Verbindungen IX sind bekannt bzw. können nach bekannten Verfahren, gegebenenfalls *in situ*, hergestellt werden. Die Herstellung der Piperazinothienobenzodiazepinone X erfolgt durch Umsetzung von Verbindungen der Formel I, worin $R^3$ —Hal bedeutet, mit entsprechenden Aminen V, das heißt Piperazin oder Homopiperazin, nach vorstehend für die Aminierung beschriebenen Methoden.

Zur Herstellung der Verbindungen I* bzw. I** werden entsprechende Ausgangsverbindungen II*, II**, III*, III**, IV*, IV**, V**, IX**, X**

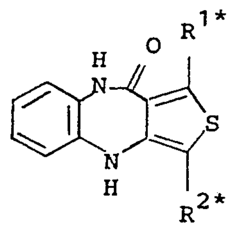 (II*), 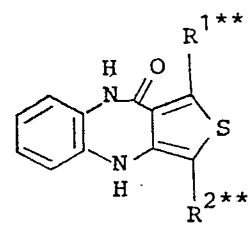 (II**)

Cl—A*—CO—Cl (III*), Cl—A**—CO—Cl (III**), (Cl—A*—CO)$_2$O (IV*), (Cl—A**—CO)$_2$O (IV**), HN($R^{4**}$)$R^{5**}$ (V**), Z**—CO—A**—N($R^{4**}$)$R^{5**}$ (IX**),

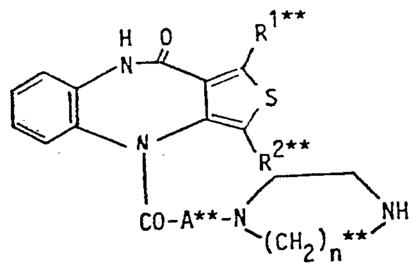 (X**)

worin

$R^{1*}$ bzw. $R^{1**}$ ein Wasserstoffatom, einen Methyl- oder Ethylrest bedeutet,

$R^{2*}$ bzw. $R^{2**}$ ein Chloratom darstellt oder eine der Bedeutungen von $R^{1*}$ bzw. $R^{1**}$ hat,

A* bzw. A** eine geradkettige oder verzweigte Alkylengruppe mit 1 oder 2 Kohlenstoffatomen bedeutet,

$R^{4**}$ einen Alkylrest mit 1 bis 4 Kohlenstoffatomen oder einen Alkenylrest mit 3 bis 4 Kohlenstoffatomen bedeutet,

$R^{5**}$ die Bedeutung von $R^{4**}$ hat oder die Gruppe —$(CH_2)_{m**}$—N($R^{6**}$)$R^{7**}$ darstellt oder

$R^{4**}$ und $R^{5**}$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl-, Ethyl- oder Benzylgruppe substituierte Piperazin-1-yl-gruppe, eine 2,4-Dimethyl-piperazin-1-yl-gruppe oder eine in 4-Position durch eine Methyl- oder Ethylgruppe substituierte Hexahydro-1H-1,4-diazepin-1-yl-gruppe bedeuten,

$R^{6**}$ eine Methyl- oder Ethylgruppe bedeutet,

$R^{7**}$ eine Methyl- oder Ethylgruppe bedeutet,

m** 2 oder 3 bedeutet,

n** 2 oder 3 bedeutet,

Z** eine Fluchtgruppe bedeutet, eingesetzt.

Die folgenden Beispiele dienen zur näheren Erläuterung der Erfindung. « F. » bedeutet « Schmelzpunkt », « Z. » bedeutet « Zersetzung ».

## Beispiel 1

3,5 g 4-Chloracetyl-9,10-dihydro-4H-thieno [3,4-b] [1,5] benzodiazepin-10-on, 8,1 g N-Methylpiperazin und 50 ml Toluol werden 2 Stunden bei 80 °C gerührt. Man versetzt mit 60 ml verdünnter Natronlauge, trennt die Schichten, schüttelt die wässerige Phase noch mehrmals mit Toluol aus und engt sie im

Vakuum zur Trockne ein. Der Rückstand wird mit wenig Aceton zur Kristallisation gebracht. Man erhält 4,2 g 9,10-Dihydro-4-[(4-methyl-piperazin-1-yl)acetyl]-4H-thieno [3,4-b] [1,5] benzodiazepin-10-on, F. 177-178 °C (Aceton).

Analog erhält man

9,10-Dihydro-3-methyl-4-[(4-methyl-piperazin-1-yl)acetyl]-4h-thieno [3,4-b] [1,5] benzodiazepin-10-on, F. 263-264 °C (Ethanol),

3-Chlor-9,10-dihydro-4-[(4-methyl-piperazin-1-yl)acetyl]-4H-thieno [3,4-b] [1,5] benzodiazepin-10-on, F. 239 °C, bzw.

9,10-Dihydro-1,3-dimethyl-4-[(4-methyl-piperazin-1-yl)-acetyl]-4H-thieno [3,4-b] [1,5] benzodiazepin-10-on, F. 204-205 °C,

durch Umsetzen von

4-Chloracetyl-9,10-dihydro-3-methyl-4H-thieno [3,4-b] [1,5] benzodiazepin-10-on,

3-Chlor-4-chloracetyl-9,10-dihydro-4H-thieno [3,4-b] [1,5] benzodiazepin-10-on bzw.

4-Chloracetyl-9,10-dihydro-1,3-dimethyl-4H-thieno [3,4-b] [1,5] benzodiazepin-10-on mit N-Methylpiperazin.

Beispiel 2

Man rührt 14,9 g 4-Chloracetyl-9,10-dihydro-4H-thieno [3,4-b] [1,5] benzodiazepin-10-on, 21 g N-Methylpiperazin und 70 ml Dioxan 1 Stunde bei 80 °C und engt die Lösung im Vakuum zur Trockne ein. Man versetzt den Rückstand mit 150 ml Isopropanol und 40 ml Wasser, tropft 25 ml konzentrierte Salzsäure zu, kühlt im Eisbad und erhält 9,10-Dihydro-4-[(4-methylpiperazin-1-yl)-acetyl]-4H-thieno [3,4-b] [1,5] benzodiazepin-10-on-dihydrochlorid im Gemisch mit N-Methylpiperazinhydrochlorid. Man löst die Hydrochloride in Wasser und Chloroform, stellt mit 2n Natronlauge auf pH 8,2, schüttelt die wässerige Phase erschöpfend mit Chloroform aus, trocknet die organische Lösung und engt sie im Vakuum zur Trockne ein. Man erhält 16 g 9,10-Dihydro-4-[(4-methylpiperazin-1-yl)acetyl]-4h-thieno [3,4-b] [1,5] benzodiazepin-10-on, F. 177-179 °C (aus Aceton).

Analog erhält man

9,10-Dihydro-4-(morpholinoacetyl)-4H-thieno [3,4-b] [1,5] benzodiazepin-10-on,

4-[(4-Benzylpiperazin-1-yl)acetyl]-9,10-dihydro-4H-thieno [3,4-b] [1,5] benzodiazepin-10-on,

4-[(4-Ethylpiperazin-1-yl)-acetyl]-9,10-dihydro-4H-thieno [3,4-b] [1,5] benzodiazepin-10-on bzw.

4-[(2,4-dimethyl-piperazin-1-yl)acetyl]-9,10-dihydro-4H-thieno [3,4-b[ [1,5] benzodiazepin-10-on

durch Umsetzung von

4-Chloracetyl-9,10-dihydro-4H-thieno [3,4-b] [1,5] benzodiazepin-10-on mit Morpholin, N-Benzylpiperazin, N-Ethylpiperazin bzw. 1,3-Dimethylpiperazin.

Beispiel 3

Man erhitzt 1,9 g 4-Chloracetyl-9,10-dihydro-4H-thieno [3,4-b] [1,5] benzodiazepin-10-on, 0,55 g Pyrrolidin, 0,85 g gemahlenes Natriumcarbonat und 15 ml absolutes Ethanol 2 Stunden zum Sieden, filtriert die heiße Lösung und engt im Vakuum ein. Man löst in Dichlormethan, wäscht die organische Lösung bei pH 7 mit Wasser, engt sie ein und erhält 1,4 g 9,10-Dihydro-4-(pyrrolidinoacetyl)-4H-thieno [3,4-b] [1,5] benzodiazepin-10-on.

Beispiel 4

Man rührt 1,9 g 4-Chloracetyl-9,10-dihydro-4H-thieno [3,4-b] [1,5] benzodiazepin-10-on, 3,7 g Piperidin und 15 ml Dioxan 1 Stunde bei 80 °C, engt im Vakuum ein und kristallisiert den Rückstand aus Isopropanol/Wasser um. Man erhält 2,0 g 9,10-Dihydro-4-(piperidin-1-yl-acetyl)-4H-thieno [3,4-b] [1,5] benzodiazepin-10-on.

Analog erhält man

9,10-Dihydro-3-methyl-4-(piperazin-1-yl-acetyl)-4H-thieno [3,4-b] [1,5] benzodiazepin-10-on (F. 225-228 °C, Z.) durch Umsetzung von 4-Chloracetyl-9,10-dihydro-3-methyl-4H-thieno [3,4-b] [1,5] benzodiazepin-10-on mit Piperazin ;

9,10-Dihydro-1,3-dimethyl-4-(morpholin-4-yl-acetyl)-4H-thieno [3,4-b] [1,5]-benzodiazepin-10-on (F. 188-190 °C) durch Umsetzung von 4-Chloracetyl-9,10-dihydro-1,3-dimethyl-4H-thieno [3,4-b] [1,5] benzodiazepin-10-on mit Morpholin ; 9,10-Dihydro-1,3-dimethyl-4-(piperidin-1-yl-acetyl)-4H-thieno [3,4-b] [1,5] benzodiazepin-10-on (F. 162-164 °C) durch Umsetzung von

4-Chloracetyl-9,10-dihydro-1,3-dimethyl-4H-thieno [3,4-b] [1,5] benzodiazepin-10-on mit Piperidin.

Beispiel 5

Man rührt 2,0 g 4-Chloracetyl-9,10-dihydro-4H-thieno [3,4-b] [1,5] benzodiazepin-10-on, 6 ml 40 %ige wässerige Dimethylaminlösung und 10 ml Dichlormethan 2 Stunden bei 35 °C, versetzt mit 0,35 g Natriumcarbonat und engt im Vakuum zur Trockne ein. Man versetzt mit wenig Wasser, schüttelt die

Lösung wiederholt mit Chloroform aus, trocknet die organische Lösung mit Natriumsulfat und engt sie zur Trockne ein. Man erhält 1,9 g 4-(Dimethylaminoacetyl)-9,10-dihydro-4H-thieno [3,4-b] [1,5] benzodiazepin-10-on.

Analog erhält man 4-(diethylaminoacetyl)-9,10-dihydro-3-methyl-4H-thieno [3,4-b] [1,5] benzodiazepin-10-on [F. 196-197 °C (aus Toluol)] bzw. 4-(Diethylaminoacetyl)-9,10-dihydro-1,3-dimethyl-4H-thieno [3,4-b] [1,5] benzodiazepin-10-on [F. 186-187 °C (aus Toluol)] durch Umsetzung von 4-Chloracetyl-9,10-dihydro-3-methyl-4H-thieno [3,4-b] [1,5] benzodiazepin-10-on bzw. 4-Chloracetyl-9,10-dihydro-1,3-dimethyl-4H-thieno [3,4-b] [1,5] benzodiazepin-10-on und Diethylamin.

## Beispiel 6

Analog Beispiel 4 erhält man
9,10-Dihydro-3-methyl-4-(pyrrolidinoacetyl)-4H-thieno [3,4-b] [1,5] benzodiazepin-10-on,
3-Chlor-9,10-dihydro-4-(pyrrolidinoacetyl)-4H-thieno [3,4-b] [1,5] benzodiazepin-10-on bzw.
9,10-Dihydro-1,3-dimethyl-4-(pyrrolidinoacetyl)-4H-thieno [3,4-b] [1,5] benzodiazepin-10-on
durch Umsetzung von
4-Chloracetyl-9,10-dihydro-3-methyl-4H-thieno [3,4-b] [1,5] benzodiazepin-10-on,
3-Chlor-4-chloracetyl-9,10-dihydro-4H-thieno [3,4-b] [1,5] benzodiazepin-10-on bzw.
4-Chloracetyl-9,10-dihydro-1,3-dimethyl-4H-thieno [3,4-b] [1,5] benzodiazepin-10-on mit Pyrrolidin.

## Beispiel 7

Analog Beispiel 5 erhält man
4-(Dimethylaminoacetyl)-9,10-dihydro-3-methyl-4H-thieno [3,4-b] [1,5] benzodiazepin-10-on,
3-Chlor-4-(dimethylaminoacetyl)-9,10-dihydro-4H-thieno [3,4-b] [1,5] benzodiazepin-10-on bzw.
4-(Dimethylaminoacetyl)-9,10-dihydro-1,3-dimethyl-4H-thieno [3,4-b] [1,5] benzodiazepin-10-on
durch Umsetzung von
4-Chloracetyl-9,10-dihydro-3-methyl-4H-thieno [3,4-b] [1,5] benzodiazepin-10-on,
3-Chlor-4-chloracetyl-9,10-dihydro-4H-thieno [3,4-b] [1,5] benzodiazepin-10-on bzw.
4-Chloracetyl-9,10-dihydro-1,3-dimethyl-4H-thieno [3,4-b] [1,5] benzodiazepin-10-on mit Dimethylamin.

## Beispiel 8

8 g 9,10-Dihydro-3-methyl-4H-thieno [3,4-b] [1,5] benzodiazepin-10-on und 5,6 ml Chloracetylchlorid in 160 ml Dioxan werden in Gegenwart von 8 g gemahlenem Kaliumcarbonat 8 Stunden unter Rückfluß gekocht. Man engt die Lösung zur Trockne ein, nimmt den Rückstand mit Toluol auf, wäscht mit Natriumbicarbonatlösung und anschließend mit Wasser und trocknet die Toluol-Lösung über Natriumsulfat. Durch Einengen erhält man 4-Chloracetyl-9,10-dihydro-3-methyl-4H-thieno [3,4-b] [1,5] benzodiazepin-10-on, F. 156-158 °C.

Analog erhält man
3-Chlor-4-chloracetyl-9,10-dihydro-4H-thieno [3,4-b] [1,5] benzodiazepin-10-on, F. 214-216 °C bzw.
4-Chloracetyl-9,10-dihydro-1,3-dimethyl-4H-thieno [3,4-b] [1,5] benzodiazepin-10-on, F. 192-195 °C,
durch Umsetzung von
3-Chlor-9,10-dihydro-4H-thieno [3,4-b] [1,5] benzodiazepin-10-on bzw.
9,10-Dihydro-1,3-dimethyl-4H-thieno [3,4-b] [1,5] benzodiazepin-10-on mit Chloracetylchlorid.

## Beispiel 9

Zu einer Suspension von 18,8 g 9,10-Dihydro-4H-thieno [3,4-b] [1,5] benzodiazepin-10-on in 500 ml Dioxan werden bei Raumtemperatur 10,4 ml Chloracetylchlorid zugetropft, wobei eine klare Lösung entsteht. Man läßt 3 Stunden stehen, engt die Lösung zur Trockne ein, nimmt den Rückstand mit Toluol auf, wäscht mit Natriumbicarbonatlösung und anschließend mit Wasser und trocknet die Toluol-Lösung über Natriumsulfat. Durch Einengen erhält man 4-Chloracetyl-9,10-dihydro-4H-thieno [3,4-b] [1,5] benzodiazepin-10-on als Öl, das langsam kristallisiert. F. 220 °C.

Analog erhält man
4-Chloracetyl-9,10-dihydro-1-methyl-4H-thieno [3,4-b] [1,5] benzodiazepin-10-on bzw. 4-Chloracetyl-9,10-dihydro-3-methyl-4H-thieno [3,4-b] [1,5] benzodiazepin-10-on, F. 156-158 °C, durch Umsetzung von 9,10-Dihydro-1-methyl-4H-thieno [3,4-b] [1,5] benzodiazepin-10-on bzw. 9,10-Dihydro-3-methyl-4H-thieno [3,4-b] [1,5] benzodiazepin-10-on mit Chloracetylchlorid.

## Beispiel 10

Zu einer siedenden Lösung von 2,2 g 9,10-Dihydro-4H-thieno [3,4-b] [1,5] benzodiazepin-10-on in 30

ml absolutem Dioxan werden in 40 Minuten gleichzeitig 2,2 g Chloracetylchlorid und 2 ml Triethylamin getropft und 3 Stunden weitergerührt Man läßt erkalten, filtriert, engt das Filtrat zur Trockne ein und chromatographiert über eine Kieselgelsäule mittels eines Gemisches von Petrolether/Essigsäureethylester (1 : 1), kristallisiert aus Toluol um und erhält 2,0 g 4-Chloracetyl-9,10-dihydro-4H-thieno [3,4-b] [1,5] benzodiazepin-10-on als Öl, das langsam kristallisiert, F. 220 °C.

Beispiel 11

Eine Lösung von 5 ml Sulfurylchlorid in 100 ml Methylenchlorid wird zu einer Lösung von 12 g 4-Chloracetyl-9,10-dihydro-4H-thieno [3,4-b] [1,5] benzodiazepin-10-on in 300 ml Methylenchlorid bei 20 °C zugetropft. Die Mischung wird 12 Stunden bei Raumtemperatur stehengelassen, dann mit einer Natriumhydrogencarbonatlösung extrahiert und mit Wasser gewaschen. Die Phase wird getrocknet und eingeengt. Der Rückstand wird mit wenig Methanol zur Kristallisation gebracht. Man erhält 7 g 3-Chlor-4-chloracetyl-9,10-dihydro-4H-thieno [3,4-b] [1,5] benzodiazepin-10-on, F. 214-216 °C (Acetonitril).

Beispiel 12

Man erwärmt ein Gemisch von 1,00 g (4-Methylpiperazin-1-yl)-essigsäure und 0,20 g 75 proz. Natriumhydrid (in Paraffinöl) in 16 ml Dimethylformamid bei 50-80 °C so lange, bis die Wasserstoffentwicklung beendet ist (2 bis 3 Stunden). Zu dem gebildeten Natriumsalz der Säure fügt man 1,35 g 9,10-Dihydro-4H-thieno [3,4-b] [1,5] benzodiazepin-10-on und tropft bei −10 °C 0,99 g 98 % Phosphoroxidchlorid in 10 Minuten zu. Man rührt 4 Stunden bei −10 °C, 4 Stunden bei 0 °C und 20 Stunden bei Raumtemperatur. Man gießt den Ansatz auf Eis, stellt mit Natronlauge auf pH 3,5, schüttelt mit Methylenchlorid aus, stellt die wässerige Phase auf pH 9 und schüttelt wieder mit Methylenchlorid aus. Man wäscht die organische Phase mit Wasser und engt sie im Vakuum ein. Man erhält 0,6 g 9,10-Dihydro-4-[(4-methyl-piperazin-1-yl) acetyl]-4H-thieno [3,4-b] [1,5] benzodiazepin-10-on, F. 177-178 °C (aus Aceton).

Analog erhält man 9,10-Dihydro-3-methyl-4-[(4-methyl-piperazin-1-yl) acetyl]-4H-thieno [3,4-b] [1,5] benzodiazepinon-10-on (F. 263-264 °C) bzw. 9,10-Dihydro-1,3-dimethyl-4-[(4-methyl-piperazin-1-yl)acetyl]-4H-thieno [3,4-b] [1,5] benzodiazepin-10-on (F. 204-205 °C) durch Umsetzung von (4-Methylpiperazin-1-yl)-essigsäure mit Natriumhydrid, 9,10-Dihydro-3-methyl-4H-thieno [3,4-b] [1,5]-benzodiazepin-10-on bzw. 9,10-Dihydro-1,3-dimethyl-4H-thieno [3,4-b] [1,5] benzodiazepin-10-on und Phosphoroxidchlorid.

Beispiel 13

Zu einer Suspension von 1,58 g (4-Methylpiperazin-1-yl)-essigsäure in 20 ml Tetrahydrofuran werden bei 0 °C 1,1 g Chlorameisensäureethylester zugetropft. Man fügt 2,18 g 9,10-Dihydro-4H-thieno [3,4-b] [1,5] benzodiazepin-10-on zu der entstandenen Suspension, rührt noch eine Stunde bei 0 °C und anschliessend 4 Stunden bei Raumtemperatur. Man gießt auf 160 ml 2 n Natronlauge, extrahiert mit Toluol und engt die organische Phase zur Trockne ein. Nach säulenchromatographischer Reinigung (Kieselgel ; Dioxan/Methanol 1 : 1) erhält man 9,10-Dihydro-4-[(4-methyl-piperazin-1-yl)-acetyl]-4H-thieno [3,4-b] [1,5] benzodiazepin-10-on, F. 177-179 °C.

Analog erhält man 9,10-Dihydro-3-methyl-4-[(4-methyl-piperazin-1-yl)-acetyl]-4H-thieno [3,4-b] [1,5] benzodiazepin-10-on (F. 263-264 °C) durch Umsetzung von 9,10-Dihydro-3-methyl-4H-thieno [3,4-b] [1,5] benzodiazepin-10-on mit (4-Methylpiperazin-1-yl)-essigsäure und Chlorameisensäureethylester.

Beispiel 14

2,0 g 9,10-Dihydro-3-methyl-4-(piperazin-1-yl-acetyl)-4H-thieno [3,4-b] [1,5] benzodiazepin-10-on, 1,3 g 98 %ige Ameisensäure und 0,3 g 35 %ige wäßrige Formaldehydlösung werden 2 Stunden auf 100-105 °C erwärmt, wobei sich die Mischung unter Gasentwicklung verflüssigt. Man engt im Vakuum ein, verdünnt mit Wasser, stellt mit verdünnter Salzsäure auf pH 3,5 und schüttelt mit Dichlormethan aus. Die wäßrige Phase wird auf pH 9 gestellt und mit Dichlormethan extrahiert. Die organische Phase wird gewaschen, getrocknet und eingeengt. Umkristallisation des Rückstands aus Methanol liefert 9,10-Dihydro-3-methyl-4-[(4-methyl-piperazin-1-yl)-acetyl]-4H-thieno [3,4-b] [1,5] benzodiazepin-10-on, F. 263-264 °C.

Die Ausgangsverbindungen erhält man auf folgende Weise :

Beispiel A : Zu einer Lösung von 21,6 g 9,10-Dihydro-4H-thieno [3,4-b] [1,5]-benzodiazepin-10-on in 300 ml Dichlormethan werden bei − 40 °C 13,5 g Sulfurylchlorid in 100 ml Dichlormethan zugetropft. Man läßt noch eine Stunde bei Raumtemperatur stehen, schüttelt mit Natriumbicarbonatlösung aus, wäscht mit Wasser, trocknet die organische Phase und engt sie ein. Den Rückstand bringt man mit wenig Methanol zur Kristallisation. Man erhält 3-Chlor-9,10-dihydro-4H-thieno [3,4-b] [1,5] benzodiazepin-10-on.

Beispiel B : 10 g 1,3,9,10-Tetrahydro-3-methyl-4H-thieno [3,4-b] [1,5] benzodiazepin-10-on und 8,2 g N-Bromsuccinimid werden in 250 ml Dimethylformamid gelöst. Nach einer Stunde wird auf 2 l Wasser gegossen. Der Niederschlag wird abgesaugt, mit heißem Toluol gelöst und mit Tonsil® geklärt. Beim

Abkühlen erhält man als Niederschlag 9,10-Dihydro-3-methyl-4H-thieno [3,4-b] [1,5] benzodiazepin-10-on, F. 228-230 °C (Methanol).

Analog erhält man

9,10-Dihydro-1-methyl-4H-thieno [3,4-b] [1,5] benzodiazepin-10-on bzw. 9,10-Dihydro-1,3-dimethyl-4H-thieno [3,4-b] [1,5] benzodiazepin-10-on (F. 195-196 °C) durch Dehydrierung von 1,3,9,10-Tetrahydro-1-methyl-4H-thieno [3,4-b] [1,5] benzodiazepin-10-on bzw. 1,3,9,10-Tetrahydro-1,3-dimethyl-4H-thieno [3,4-b] [1,5] benzodiazepin-10-on mit N-Bromsuccinimid.

Beispiel C : 49,9 g o-Phenylendiamin und 80 g 5-Methyl-tetrahydro-4-oxo-3-thiophencarbonsäure werden in 4,5 l Toluol gekocht. Im Verlauf von 7 Stunden wird das entstandene Wasser mit 2 l des Lösungsmittels azeotrop abdestilliert. Das Lösungsmittel wird entfernt. Man erhält 1,3,9,10-Tetrahydro-3-methyl-4H-thieno [3,4-b] [1,5] benzodiazepin-10-on, F. 195-197 °C (Isopropanol).

Analog erhält man

1,3,9,10-Tetrahydro-1-methyl-4H-thieno [3,4-b] [1,5] benzodiazepin-10-on bzw.

1,3,9,10-Tetrahydro-1,3-dimethyl-4H-thieno [3,4-b] [1,5] benzodiazepin-10-on (F. 148-150 °C) durch Umsetzung von Tetrahydro-2-methyl-4-oxo-3-thiophencarbonsäure bzw. Tetrahydro-2,5-dimethyl-4-oxo-3-thiophencarbonsäure mit o-Phenylendiamin.

Die Folgenden Beispiele beschreiben die Formulierung der erfingungsgemäßen Verbindung zu Arzneimitteln.

## Beispiel 15

10 000 Tabletten mit einem Wirkstoffgehalt von 20 mg werden aus folgenden Bestandteilen hergestellt :

200 g 9,10-Dihydro-4-[(4-methyl-piperazin-1-yl)acetyl]-4H-thieno [3,4-b] [1,5] benzodiazepin-10-on, 900 g Maisstärke, 500 g Milchzucker, 30 g amorphe Kieselsäure und 40 g Natriumlaurylsulfat werden gemischt und gesiebt. Diese Mischung wird mit einer Lösung von 50 g Polyvinylpyrrolidon (Mittl. Mol.-Gewicht 25 000) in 320 ml Alkohol befeuchtet und durch ein Sieb der Maschenweite von 1,25 mm granuliert. Das Granulat wird bei 40° getrocknet und mit 160 g Pektin, 100 g Talk und 20 g Magnesiumstearat gemischt. Diese Mischung wird zu Tabletten à 200 mg mit 8 mm Durchmesser verpreßt.

## Beispiel 16

100 000 Kapseln mit einem Wirkstoffgehalt von 30 mg werden aus folgenden Bestandteilen hergestellt :

3 000 g 9,10-Dihydro-4-[(4-methyl-piperazin-1-yl)acetyl]-4H-thieno [3,4-b] [1,5] benzodiazepin-10-on werden mit 5 000 g Neutrolöl (Miglyol® 812) gemischt und in Weichgelatinekapseln abgefüllt.

## Beispiel 17

100 000 Kapseln mit einem Wirkstoffgehalt von 30 mg werden aus folgenden Bestandteilen hergestellt :

1 500 g 9,10-Dihydro-4-[(4-methyl-piperazin-1-yl)acetyl]-4H-thieno [3,4-b] [1,5] benzodiazepin-10-on und 1 500 g Magnesiumtrisilikat werden mit 5 000 g Neutralöl (Miglyol® 812) gemischt und in Weichgelatinekapseln abgefüllt.

Analog Beispiel 15 bis 17 werden entsprechende Arzneimittel erhalten, indem 9,10-Dihydro-3-methyl-4-[(4-methyl-piperazin-1-yl)acetyl]-4H-thieno [3,4-b] [1,5] benzodiazepin-10-on bzw. 9,10-Dihydro-1,3-dimethyl-4-[(4-methyl-piperazin-1-yl)acetyl]-4H-thieno [3,4-b] [1,5] benzodiazepin-10-on anstelle von 9,10-Dihydro-4-[(4-methyl-piperazin-1-yl)acetyl]-4H-thieno [3,4-b] [1,5] benzodiazepin-10-on verwendet werden.

## Pharmakologie

Die ausgezeichnete Magenschutzwirkung der pharmakologisch wirksamen substituierten Thienobenzodiazepinone läßt sich am Modell der sogenannten Shay-Ratte nachweisen. Hierbei erweisen sich die erfindungsgemäßen Verbindungen dem bekannten Handelsprodukt Carbenoxolon (1) in der Magenschutzwirkung und der therapeutischen Breite eindeutig überlegen, wie sich beispielsweise am Vergleich von (1) und 9,10-Dihydro-4-[(4-methyl-piperazin-1-yl)acetyl]-4H-thieno [3,4-b] [1,5] benzodiazepin-10-on (2), 3-Chlor-9,10-dihydro-4-[(4-methylpiperazin-1-yl)acetyl]-4H-thieno [3,4-b] [1,5] benzodiazepin-10-on (3), 9,10-Dihydro-3-methyl-4-[(4-methylpiperazin-1-yl)acetyl]-4H-thieno [3,4-b] [1,5] benzodiazepin-10-on (4) und 9,10-Dihydro-1,3-dimethyl-4-[(4-methylpiperazin-1-yl)acetyl]-4H-thieno [3,4-b] [1,5] benzodiazepin-10-on (5) zeigen läßt.

Tabelle I

Antiulcerogene Wirkung und Toxizität von Thienobenzodiazepinonen

| Lfd.Nr. | Toxizität $LD_{50}$[mg/kg]i.v. Maus | Magenschutzwirkung $ED_{50}$[mg/kg]p.o. Ratte | TQ $LD_{50}/ED_{50}$ | Magensekretion+) % Hemmung Ratte |
|---|---|---|---|---|
| 1 | 290 | ~ 70 | 4,1 | 7 |
| 2 | 190 | 2,5 | 76 | 35 |
| 3 | 75 | ≲1 | ≳75 | 28 |
| 4 | 130 | ~ 0,3 | ~433 | 20 |
| 5 | 180 | 1,3 | 139 | 17 |

$ED_{50}$ = Dosis, die den Ulcusindex bei der behandelten Gruppe gegenüber der Kontrollgruppe um 50 % mindert
$LD_{50}$ = Dosis, bei der 50 % der Tiere sterben
TQ = Therapeutischer Quotient $LD_{50}/ED_{50}$
+) % Hemmung = Hemmung der Magensekretion (in %) 4 Stunden nach Applikation der antiulcerogenen $ED_{50}$

Es sei besonders darauf hingewiesen, daß bei Verbindung 1 zwar eine $ED_{50}$ noch bestimmbar ist, die Dosiswirkungskurve dann jedoch sehr stark abflacht, so daß selbst bei 300 mg/kg keine wesentliche Steigerung der antiulcerogenen Wirkung erreichbar ist. Im Gegensatz dazu ist die Wirkung der Verbindungen 2 bis 5 streng dosisabhängig ; es lassen sich Hemmeffekte bis 95 % erreichen.

Die Prüfung der antiulcerogenen Wirkung erfolgte nach der Methode der sogenannten Shay-Ratte :

Die Ulcusprovokation erfolgt bei 24 Stunden nüchtern gehaltenen Ratten (weiblich, 180-200 g, 4 Tiere je Käfig auf hohem Gitterrost) durch Pylorusligatur (unter Diethylethernarkose) und orale Applikation von 100 mg/10 ml/kg Acetylsalicylsäure. Die zu testenden Substanzen werden oral (10 ml/kg) 1 Stunde vor der Pylorusligatur verabreicht. Der Wundverschluß wird mittels Michelklammern vorgenommen. 4 Stunden danach erfolgt die Tötung der Tiere im Etherrausch durch Atlas-Dislokation und die Resektion des Magens. Der Magen wird längs eröffnet und auf einer Korkplatte fixiert, nachdem zuvor die Menge des sezernierten Magensaftes (Volumen) bestimmt wurde ; mit einem Stereomikroskop werden bei 10-facher Vergrößerung Anzahl und Größe (= Durchmesser) vorhandener Ulcera ermittelt. Das Produkt aus Schweregrad (Gemäß nachfolgender Punkteskala) und Anzahl der Ulcera dient als individueller Ulcusindex.

Punkteskala :
Keine Ulcera 0
Ulcusdurchmesser 0,1-1,4 mm 1
1,5-2,4 mm 2
2,5-3,4 mm 3
3,5-4,4 mm 4
4,5-5,4 mm 5
> 5,5 mm 6

Als maß für den antiulcerogenen Effekt dient die Minderung des mittleren Ulcus-Index jeder behandelten Gruppe gegenüber dem der Kontrollgruppe (= 100 %). Die $ED_{50}$ bezeichnet die Dosis, die den mittleren Ulcusindex um 50 % mindert.

Bestimmung der Toxizität

Die Toxizitätsuntersuchungen werden an weiblichen NMRI-Mäusen (Körpergewicht 22-26 g) durchgeführt. Die Tiere (5 Tiere pro Dosis) erhalten Futter und wasser ad libitum. Verschiedene Dosen der Substanzen werden intravenös (Injektionsdauer 1 Minute) verabreicht. Die Beobachtungsdauer beträgt 7 Tage. Die $LD_{50}$, d. h. die Dosis, bei der 50 % der Tiere sterben wird mittels linearer Regression ermittelt.

**Ansprüche** (für die Vertragsstaaten : BE, CH, DE, FR, GB, IT, LI, LU, NL, SE)

1. Substituierte Thienobenzodiazepinone der allgemeinen Formel I

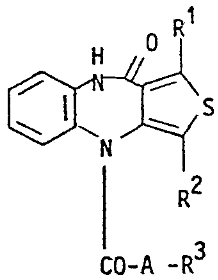

(I)

worin

$R^1$ ein Wasserstoffatom oder einen Alkylrest mit 1 bis 4 Kohlenstoffatomen bedeutet,

$R^2$ ein Halogenatom darstellt oder eine der Bedeutungen von $R^1$ hat,

$R^3$ ein Halogenatom oder die Gruppe $-N(R^4)R^5$ und

$R^4$ einen Alkylrest mit 1 bis 4 Kohlenstoffatomen oder einen Alkenylrest mit 3 bis 5 Kohlenstoffatomen bedeuten,

$R^5$ eine der Bedeutungen von $R^4$ hat oder die Gruppe $-(CH_2)_m-N(R^6)R^7$ darstellt oder

$R^4$ und $R^5$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl-, Ethyl- oder Benzylgruppe substituierte Piperazin-1-yl-gruppe, eine 2,4-Dimethylpiperazin-1-yl-gruppe oder eine in 4-Position durch eine Methyl- oder Ethylgruppe substituierte Hexahydro-1H-1,4-diazepin-1-yl-gruppe bedeuten und

$R^6$ eine Alkylgruppe mit 1 bis 4 Kohlenstoffatomen

$R^7$ eine Alkylgruppe mit 1 bis 4 Kohlenstoffatomen,

A eine geradkettige oder verzweigte Alkylengruppe mit 1 bis 5 Kohlenstoffatomen und

m 2 oder 3 bedeuten,

sowie ihre Säureadditionssalze.

2. Substituierte Thienobenzodiazepinone nach Anspruch 1, gekennzeichnet durch die allgemeine Formel I*

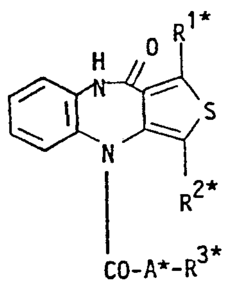

(I*)

worin

$R^{1*}$ ein Wasserstoffatom, einen Methyl- oder Ethylrest bedeutet,

$R^{2*}$ ein Chloratom darstellt oder eine der Bedeutungen von $R^{1*}$ hat,

$R^{3*}$ ein Chloratom und

A* eine geradkettige oder verzweigte Alkylengruppe mit 1 oder 2 Kohlenstoffatomen bedeuten.

3. Substituierte Thienobenzodiazepinone nach Anspruch 1, gekennzeichnet durch die allgemeine Formel I**

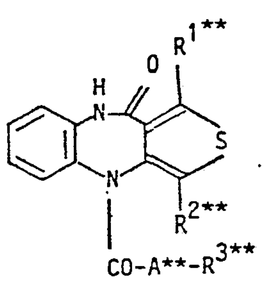

(I**)

worin

$R^{1**}$ ein Wasserstoffatom, einen Methyl- oder Ethylrest bedeutet,

$R^{2**}$ ein Chloratom darstellt oder eine der Bedeutungen von $R^{1**}$ hat,

$R^{3**}$ die Gruppe $-N(R^{4**})R^{5**}$ und

$R^{4**}$ einen Alkylrest mit 1 bis 4 Kohlenstoffatomen oder einen Alkenylrest mit 3 bis 4 Kohlenstoffatomen bedeuten,

$R^{5**}$ die Bedeutung von $R^{4**}$ hat oder die Gruppe $-(CH_2)_{m**}-N(R^{6**})R^{7**}$ darstellt oder

$R^{4**}$ und $R^{5**}$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl-, Ethyl- oder Benzylgruppe substituierte Piperazin-1-yl-gruppe, eine 2,4-Dimethyl-piperazin-1-yl-gruppe oder eine in 4-Position durch eine Methyl- oder Ethylgruppe substituierte Hexahydro-1H-1,4-diazepin-1-yl-gruppe bedeuten und

$R^{6**}$ eine Methyl- oder Ethylgruppe,

$R^{7**}$ eine Methyl- oder Ethylgruppe,

m** 2 oder 3,

A** eine geradkettige oder verzweigte Alkylengruppe mit 1 oder 2 Kohlenstoffatomen bedeuten, und ihre Säureadditionssalze.

4. Verbindungen nach Anspruch 3, in denen $R^{1**}$ ein Wasserstoffatom, einen Methyl- oder Ethylrest bedeutet, $R^{2**}$ ein Chloratom darstellt oder eine der Bedeutungen von $R^{1**}$ hat; $R^{3**}$ die Gruppe $-N(R^{4**})R^{5**}$ darstellt, $R^{4**}$ einen Methyl- oder Ethylrest bedeutet, $R^{5**}$ die Bedeutung von $R^{4**}$ hat oder die Gruppe $(CH_2)_{m**}-N(R^{6**})R^{7**}$ darstellt oder $R^{4**}$ und $R^{5**}$ gemeinsam unter Einschluß des Stickstoffatoms einen Pyrrolidino-, Piperidino- oder Hexahydroazepin-1-yl-rest, $R^{6**}$ und $R^{7**}$ einen Methyl- oder Ethylrest, m** 2 und A** eine Methylengruppe bedeuten, sowie ihre pharmakologisch verträglichen Säureadditionssalze.

5. Verbindungen nach Anspruch 3, in denen $R^{1**}$ ein Wasserstoffatom, einen Methyl- oder Ethylrest bedeutet, $R^{2**}$ ein Chloratom darstellt oder eine der Bedeutungen von $R^{1**}$ hat; $R^{3**}$ die Gruppe $-N(R^{4**})R^{5**}$ darstellt, $R^{4**}$ und $R^{5**}$ gemeinsam unter Einschluß des Stickstoffatoms eine in 4-Stellung durch eine Methyl-, Ethyl- oder Benzylgruppe substituierte Piperazin-1-yl-gruppe, eine 2,4-Dimethylpiperazin-1-yl-gruppe oder eine in 4-Stellung durch eine Methyl- oder Ethylgruppe substituierte hexahydro-1H-1,4-diazepin-1-yl-gruppe und $A^{**}$ eine Methylengruppe bedeuten, sowie ihre pharmakologisch verträglichen Säureadditionssalze.

6. Verbindungen nach Anspruch 3, in denen $R^{1**}$ ein Wasserstoffatom oder einen Methylrest, $R^{2**}$ ein Wasserstoffatom oder einen Methylrest darstellt, $R^{3**}$ die Gruppe $-N(R^{4**})R^{5**}$ darstellt, $R^{4**}$ und $R^{5**}$ gemeinsam unter Einschluß des Stickstoffatoms eine in 4-Stellung durch eine Methylgruppe substituierte Piperazin-1-yl-gruppe und A** eine Methylengruppe bedeuten, sowie ihre pharmakologisch verträglichen Säureadditionssalze.

7. Arzneimittel, die ein oder mehrere Thienobenzodiazepinone der allgemeinen Formel Ia

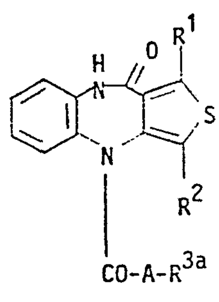

(Ia)

worin

$R^1$ ein Wasserstoffatom oder einen Alkylrest mit 1 bis 4 Kohlenstoffatomen bedeutet,

$R^2$ ein Halogenatom darstellt oder eine der Bedeutungen von $R^1$ hat,

$R^{3a}$ die Gruppe $-N(R^4)R^5$ und

$R^4$ einen Alkylrest mit 1 bis 4 Kohlenstoffatomen oder einen Alkenylrest mit 3 bis 5 Kohlenstoffatomen bedeuten,

$R^5$ eine der Bedeutungen von $R^4$ hat oder die Gruppe $-(CH_2)_m-N(R^6)R^7$ darstellt oder

$R^4$ und $R^5$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl-, Ethyl- oder Benzylgruppe substituierte Piperazin-1-yl-gruppe, eine 2,4-Dimethylpiperazin-1-yl-gruppe oder eine in 4-Position durch eine Methyl- oder Ethylgruppe substituierte Hexahydro-1H-1,4-diazepin-1-yl-gruppe bedeuten und

$R^6$ eine Alkylgruppe mit 1 bis 4 Kohlenstoffatomen,

$R^7$ eine Alkylgruppe mit 1 bis 4 Kohlenstoffatomen,

A eine geradkettige oder verzweigte Alkylengruppe mit 1 bis 5 Kohlenstoffatomen und

m 2 oder 3 bedeuten,

und/oder ihre pharmakologisch verträglichen Säureadditionssalze enthalten.

8. Verfahren zur Herstellung der substituierten Thienobenzodiazepinone der allgemeinen Formel I

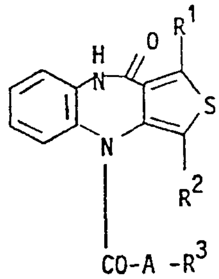

(I)

worin

$R^1$ ein Wasserstoffatom oder einen Alkylrest mit 1 bis 4 Kohlenstoffatomen bedeutet,

$R^2$ ein Halogenatom darstellt oder eine der Bedeutungen von $R^1$ hat,

$R^3$ ein Halogenatom oder die Gruppe $—N(R^4)R^5$ und

$R^4$ einen Alkylrest mit 1 bis 4 Kohlenstoffatomen oder einen Alkenylrest mit 3 bis 5 Kohlenstoffatomen bedeuten,

$R^5$ eine der Bedeutungen von $R^4$ hat oder die Gruppe $—(CH_2)_m—N(R^6)R^7$ darstellt oder

$R^4$ und $R^5$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl-, Ethyl- oder Benzylgruppe substituierte Piperazin-1-yl-gruppe, eine 2,4-Dimethylpiperazin-1-yl-gruppe oder eine in 4-Position durch eine Methyl- oder Ethylgruppe substituierte Hexahydro-1H-1,4-diazepin-1-yl-gruppe bedeuten und

$R^6$ eine Alkylgruppe mit 1 bis 4 Kohlenstoffatomen,

$R^7$ eine Alkylgruppe mit 1 bis 4 Kohlenstoffatomen,

A eine geradkettige oder verzweigte Alkylengruppe mit 1 bis 5 Kohlenstoffatomen und

m 2 oder 3 bedeuten,

sowie ihrer Säureadditionssalze, dadurch gekennzeichnet, daß man Thienobenzodiazepinone der allgemeinen Formel II

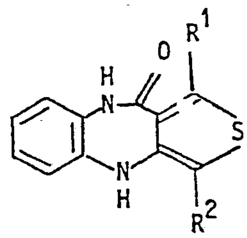

(II)

worin

$R^1$ und $R^2$ die oben angegebene Bedeutung haben, acyliert und gegebenenfalls anschließend aminiert und/oder erhaltenen Basen in die Säureadditionssalze oder erhaltene Säureadditionssalze in die freie Base oder pharmakologisch verträgliche Säureadditionssalze überführt.

9. Verfahren nach Anspruch 8, dadurch gekennzeichnet, daß man die Acylierung mit Verbindungen der allgemeinen Formeln Hal—A—CO—Hal′ (III) oder $(Hal—A—CO)_2O$ (IV), worin Hal und Hal′ ein Halogenatom und A eine Alkylengruppe mit 1 bis 5 Kohlenstoffatomen bedeuten, durchführt.

10. Verfahren nach Anspruch 8, dadurch gekennzeichnet, daß man bei der sich gegebenenfalls anschließenden Aminierung Verbindungen der allgemeinen Formel I, worin $R^3$ ein Halogenatom darstellt, und sekundäre Amine der allgemeinen Formel $HN(R^4)R^5$ (V), worin $R^4$ und $R^5$ die in Anspruch 8 angegebene Bedeutung haben, einsetzt.

11. Verfahren nach Anspruch 10, dadurch gekennzeichnet, daß man bei der sich gegebenenfalls anschließenden Aminierung als sekundäre Amine V Piperazin oder Homopiperazin einsetzt und das erhaltene Reaktionsprodukt der allgemeinen Formel X

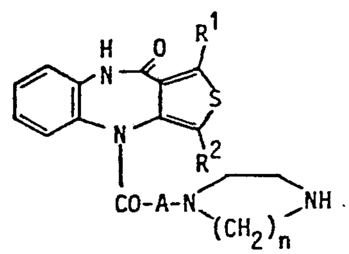

. (X)

worin $R^1$, $R^2$ und A die in Anspruch 8 angegebene Bedeutung haben und n 2 oder 3 bedeutet, methyliert oder ethyliert.

12. Verfahren nach Anspruch 8, dadurch gekennzeichnet, daß man die Acylierung mit Verbindungen der allgemeinen Formel $Z—CO—A—N(R^4)R^5$ (IX), worin A, $R^4$ und $R^5$ die in Anspruch 8 angegebene Bedeutungen haben und Z eine Fluchtgruppe darstellt, durchführt.

13. Substituierte Thienobenzodiazepinone der allgemeinen Formel Ia, worin die Substituenten die in Anspruch 7 angegebene Bedeutung haben, zur Anwendung bei der Behandlung und Prophylaxe von Krankheiten, die auf Erkrankungen des Magens oder Darms beruhen.

14. Verbindung nach Anspruch 3, in der $R^{1**}$ ein Wasserstoffatom, $R^{2**}$ einen Methylrest, $R^{4**}$ und $R^{5**}$ gemeinsam unter Einschluß des Stickstoffatoms eine in 4-Stellung durch eine Methylgruppe substituierte Piperazin-1-yl-gruppe und A** eine Methylengruppe bedeuten, sowie ihre pharmakologisch verträglichen Säureadditionssalze.

**Ansprüche** (für den Vertragsstaat AT)

1. Verfahren zur Herstellung von substituierten Thienobenzodiazepinonen der allgemeinen Formel I

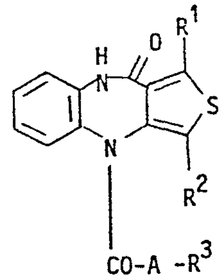

(I)

worin

$R^1$ ein Wasserstoffatom oder einen Alkylrest mit 1 bis 4 Kohlenstoffatomen bedeutet,

$R^2$ ein Halogenatom darstellt oder eine der Bedeutungen von $R^1$ Hat,

$R^3$ ein Halogenatom oder die Gruppe $—N(R^4)R^5$ und

$R^4$ einen Alkylrest mit 1 bis 4 Kohlenstoffatomen oder einen Alkenylrest mit 3 bis 5 Kohlenstoffatomen bedeuten,

$R^5$ eine der Bedeutungen von $R^4$ hat oder die Gruppe $—(CH_2)_m—N(R^6)R^7$ darstellt oder

$R^4$ und $R^5$ gemeinsam unter Einschluß des Stockstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl-, Ethyl- oder Benzylgruppe substituierte Piperazin-1-yl-gruppe, eine 2,4-Dimethylpiperazin-1-yl-gruppe oder eine in 4-Position durch eine Methyl- oder Ethylgruppe substituierte Hexahydro-1 H-1,4-diazepin-1-yl-gruppe bedeuten und

$R^6$ eine Alkylgruppe mit 1 bis 4 Kohlenstoffatomen,

$R^7$ eine Alkylgruppe mit 1 bis 4 Kohlenstoffatomen,

A eine geradkettige oder verzweigte Alkylengruppe mit 1 bis 5 Kohlenstoffatomen und

m 2 oder 3 bedeuten,

sowie ihren Säureadditionssalzen, dadurch gekennzeichnet, daß man Thienobenzodiazepinone der allgemeinen Formel II

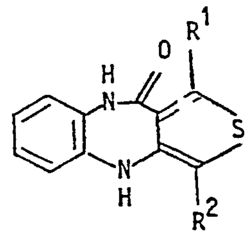

(II)

worin $R^1$ und $R^2$ die oben angegebene Bedeutung haben, acyliert und gegebenenfalls anschließend aminiert und/oder erhaltene Basen in die Säureadditionssalze oder erhaltene Säureadditionssalze in die freie Base oder pharmakologisch verträgliche Säureadditionssalze überführt.

2. Verfahren nach Anspruch 1, dadurch gekennzeichnet, daß man die Acylierung mit Verbindungen der allgemeinen Formeln Hal—A—CO—Hal' (III) oder $(Hal—A—CO)_2O$ (IV), worin Hal und Hal' ein Halogenatom und A eine Alkylengruppe mit 1 bis 5 Kohlenstoffatomen bedeuten, durchführt.

3. Verfahren nach Anspruch 1, dadurch gekennzeichnet, daß man bei der sich gegebenenfalls anschließenden Aminierung Verbindungen der allgemeinen Formel I, worin $R^3$ ein Halogenatom darstellt,

und sekundäre Amine der allgemeinen Formel $HN(R^4)R^5$ (V), worin $R^4$ und $R^5$ die in Anspruch 1 angegebene bedeutung haben, einsetzt.

4. Verfahren nach Anspruch 3, dadurch gekennzeichnet, daß man bei der sich gegebenenfalls anschließenden Aminierung als sekundäre Amine V Piperazin oder Homopiperazin einsetzt und das erhaltene Reaktionsprodukt der allgemeinen Formel X

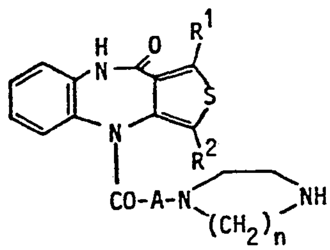

(X)

worin $R^1$, $R^2$ und A die in Anspruch 1 angegebene Bedeutung haben und n 2 oder 3 bedeutet, methyliert oder ethyliert.

5. Verfahren nach Anspruch 1, dadurch gekennzeichnet, daß man die Acylierung mit Verbindungen der allgemeinen Formel $Z—CO—A—N(R^4)R^5$ (IX), worin A, $R^4$ und $R^5$ die in Anspruch 1 angegebenen Bedeutungen haben und Z eine Fluchtgruppe darstellt, durchführt.

6. Verfahren nach Anspruch 1 und 2, dadurch gekennzeichnet, daß man substituierte Thienobenzodiazepinone der allgemeinen Formel I*

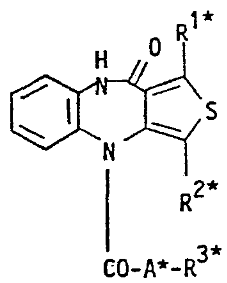

(I*)

worin

$R^{1*}$ ein Wasserstoffatom, einen Methyl- oder Ethylrest bedeutet,

$R^{2*}$ ein Chloratom darstellt oder eine der Bedeutungen von $R^{1*}$ hat,

$R^{3*}$ ein Chloratom und

A* eine geradkettige oder verzweigte Alkylengruppe mit 1 oder 2 Kohlenstoffatomen bedeuten, durch Umsetzung von Thienobenzodiazepinonen der allgemeinen Formel II*

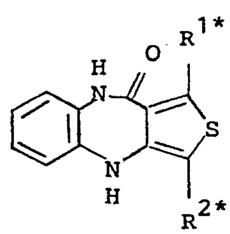

(II*)

worin $R^{1*}$ und $R^{2*}$ die oben angegebene Bedeutung haben, mit Säurederivaten III* Cl—A*—CO—Cl (III*) oder IV* $(Cl—A^*—CO)_2O$ (IV*), worin A* die oben angegebene Bedeutung hat, herstellt.

7. Verfahren nach Anspruch 1 und 3, dadurch gekennzeichnet, daß man substituierte Thienobenzodiazepinone der allgemeinen Formel I**

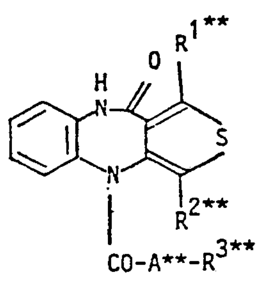

(I**)

worin

$R^{1**}$ ein Wasserstoffatom, einen Methyl- oder Ethylrest bedeutet,

$R^{2**}$ ein Chloratom darstellt oder eine der Bedeutungen von $R^{1**}$ hat,

$R^{3**}$ die Gruppe $-N(R^{4**})R^{5**}$ und

$R^{4**}$ einen Alkylrest mit 1 bis 4 Kohlenstoffatomen oder einen Alkenylrest mit 3 bis 4 Kohlenstoffatomen bedeuten,

$R^{5**}$ die Bedeutung von $R^{4**}$ hat oder die Gruppe $-(CH_2)_{m**}-N(R^{6**})R^{7**}$ darstellt oder

$R^{4**}$ und $R^{5**}$ gemeinsam unter Einschluß des Stickstoffatoms, an das sie gebunden sind, eine Morpholinogruppe, eine Pyrrolidinogruppe, eine Piperidinogruppe, eine Hexahydroazepin-1-yl-gruppe, eine gegebenenfalls in 4-Position durch eine Methyl-, Ethyl- oder Benzylgruppe substituierte Piperazin-1-yl-gruppe, eine 2,4-Dimethyl-piperazin-1-yl-gruppe oder eine in 4-Position durch eine Methyl- oder Ethylgruppe substituierte Hexahydro-1H-1,4-diazepin-1-yl-gruppe bedeuten und

$R^{6**}$ eine Methyl- oder Ethylgruppe,

$R^{7**}$ eine Methyl- oder Ethylgruppe,

m** 2 oder 3,

A** eine geradkettige oder verzweigte Alkylengruppe mit 1 oder 2 Kohlenstoffatomen bedeuten, und ihre Säureadditionssalze durch Umsetzung von Verbindungen der allgemeinen Formel I**, worin $R^{3**}$ ein Chloratom bedeutet, mit Aminen der allgemeinen Formel V** $HN(R^{4**})\ R^{5**}$ (V**), worin $R^{4**}$ und $R^{5**}$ die oben angegebene Bedeutung haben, herstellt.

8. Verfahren nach Anspruch 7, dadurch gekennzeichnet, daß man Verbindungen der allgemeinen Formel I**, in der $R^{1**}$ ein Wasserstoffatom, einen Methyl- oder Ethylrest bedeutet, $R^{2**}$ ein Chloratom darstellt oder eine der Bedeutungen von $R^{1**}$ hat; $R^{3**}$ die Gruppe $-N(R^{4**})R^{5**}$ darstellt, $R^{4**}$ einen Methyl- oder Ethylrest bedeutet, $R^{5**}$ die Bedeutung von $R^{4**}$ hat oder die Gruppe $-(CH_2)_{m**}-N(R^{6**})R^{7**}$ darstellt oder $R^{4**}$ und $R^{5**}$ gemeinsam unter Einschluß des Stickstoffatoms einen Pyrrolidino-, Piperidino- oder Hexahydroazepin-1-yl-rest, $R^{6**}$ und $R^{7**}$ einen Methyl- oder Ethylrest, m** 2 und A** eine Methylengruppe bedeuten, sowie ihre pharmakologisch verträglichen Säureadditionssalze herstellt.

9. Verfahren nach Anspruch 7, dadurch gekennzeichnet, daß man Verbindungen der allgemeinen Formel I**, in der $R^{1**}$ ein Wasserstoffatom, einen Methyl- oder Ethylrest bedeutet, $R^{2**}$ ein Chloratom darstellt oder eine der Bedeutungen von $R^{1**}$ hat; $R^{3**}$ die Gruppe $-N(R^{4**})R^{5**}$ darstellt, $R^{4**}$ und $R^{5**}$ gemeinsam unter Einschluß des Stickstoffatoms eine gegebenenfalls in 4-Stellung durch eine Methyl-, Ethyl- oder Benzylgruppe substituierte Piperazin-1-yl-gruppe, eine 2,4-Dimethyl-piperazin-1-yl-gruppe oder eine in 4-Stellung durch eine Methyl- oder Ethylgruppe substituierte hexahydro-1H-1,4-diazepin-1-yl-gruppe und A** eine Methylengruppe bedeuten, sowie ihre pharmakologisch verträglichen Säureadditionssalze herstellt.

10. Verfahren nach Anspruch 7, dadurch gekennzeichnet, daß man Verbindungen der allgemeinen Formel I**, in der $R^{1**}$ ein Wasserstoffatom oder einen Methylrest, $R^{2**}$ ein Wasserstoffatom oder einen Methylrest bedeutet, $R^{3**}$ die Gruppe $-N(R^{4**})R^{5**}$ darstellt, $R^{4**}$ und $R^{5**}$ gemeinsam unter Einschluß des Stickstoffatoms eine in 4-Stellung durch eine Methylgruppe substituierte Piperazin-1-yl-gruppe und A** eine Methylengruppe bedeuten, sowie ihre pharmakologisch verträglichen Säureadditionssalze herstellt.

11. Verfahren nach Anspruch 1 und 4, dadurch gekennzeichnet, daß man substituierte Thienobenzodiazepinone der allgemeinen Formel I**

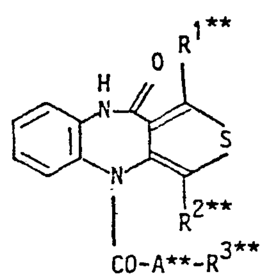

(I**)

worin

$R^{1**}$ ein Wasserstoffatom, einen Methyl- oder Ethylrest bedeutet,

$R^{2**}$ ein Chloratom darstellt oder eine der Bedeutungen von $R^{1**}$ hat,

$R^{3**}$ die Gruppe $-N(R^{4**})R^{5**}$,

$R^{4**}$ und $R^{5**}$ gemeinsam unter Einschluß des Stickstoffatoms, an das sie gebunden sind, eine in 4-Position durch eine Methyl- oder Ethylgruppe substituierte Piperazin-1-yl-gruppe oder eine in 4-Position durch eine Methyl- oder Ethylgruppe substituierte Hexahydro-1H-1,4-diazepin-1-yl-gruppe bedeuten,

A** eine geradkettige oder verzweigte Alkylengruppe mit 1 oder 2 Kohlenstoffatomen bedeuten, und ihre Säureadditionssalze durch Umsetzung von Verbindungen der allgemeinen Formel I**, worin

$R^{3**}$ ein Chloratom bedeutet, mit Piperazin oder Homopiperazin als Aminen der allgemeinen Formel V** und Methylierung oder Ethylierung der erhaltenen Piperazinothienobenzodiazepinone der allgemeinen Formel X**

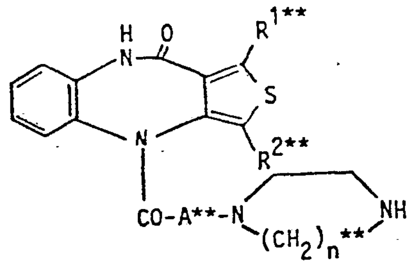

(X**)

worin $R^{1**}$, $R^{2**}$ und A** die vorstehend angegebene Bedeutung haben und n** 2 oder 3 bedeutet, herstellt.

12. Verfahren nach Anspruch 11, dadurch gekennzeichnet, daß man Verbindungen der allgemeinen Formel I**, in der $R^{1**}$ ein Wasserstoffatom oder einen Methylrest bedeutet, $R^{2**}$ eine der Bedeutungen von $R^{1**}$ hat; $R^{3**}$ die Gruppe —N($R^{4**}$)$R^{5**}$ darstellt, $R^{4**}$ und $R^{5**}$ gemeinsam unter Einschluß des Stickstoffatoms eine in 4-Stellung durch eine Methylgruppe substituierte Piperazin-1-yl-gruppe und A** eine Methylengruppe bedeuten, sowie ihre pharmakologisch verträglichen Säureadditionssalze herstellt.

13. Verfahren nach Anspruch 1 und 5, dadurch gekennzeichnet, daß man substituierte Thienobenzodiazepinone der allgemeinen Formel I**

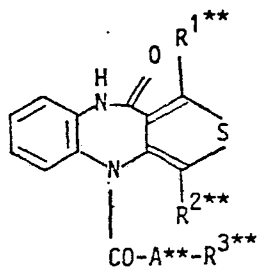

(I**)

worin $R^{1**}$, $R^{2**}$, $R^{3**}$ und A** die in Anspruch 7 angegebene Bedeutung haben, durch Umsetzung von Thienobenzodiazepinonen der allgemeinen Formel II**

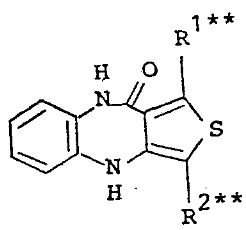

(II**)

worin $R^{1**}$ und $R^{2**}$ die vorstehend angegebene Bedeutung haben, mit reaktiven Säurederivaten der allgemeinen Formel IX** Z**—CO—A**—N—($R^{4**}$)$R^{5**}$ (IX**), worin Z** eine Fluchtgruppe darstellt und A**, $R^{4**}$ und $R^{5**}$ die in Anspruch 7 angegebene Bedeutung haben, herstellt.

14. Verfahren nach Anspruch 7, dadurch gekennzeichnet, daß man die Verbindung der allgemeinen Formel I**, in der $R^{1**}$ ein Wasserstoffatom, $R^{2**}$ einen Methylrest, $R^{3**}$ die Gruppe —N($R^{4**}$)$R^{5**}$, $R^{4**}$ und $R^{5**}$ gemeinsam unter Einschluß des Stickstoffatoms eine in 4-Stellung durch eine Methylgruppe substituierte Piperazin-1-yl-gruppe und A** eine Methylengruppe bedeuten, sowie ihre pharmakologisch verträglichen Säureadditionssalze herstellt.

15. Verfahren nach Anspruch 11, dadurch gekennzeichnet, daß man die Verbindung der allgemeinen Formel I**, in der $R^{1**}$ ein Wasserstoffatom, $R^{2**}$ einen Methylrest, $R^{3**}$ die Gruppe —N($R^{4**}$)$R^{5**}$, $R^{4**}$ und $R^{5**}$ gemeinsam unter Einschluß des Stickstoffatoms eine in 4-Stellung durch eine Methylgruppe substituierte Piperazin-1-yl-gruppe und A** eine Methylengruppe bedeuten, sowie ihre pharmakologisch verträglichen Säureadditionssalze herstellt.

16. Verfahren nach Anspruch 13, dadurch gekennzeichnet, daß man die Verbindung der allgemeinen Formel I**, in der $R^{1**}$ ein Wasserstoffatom, $R^{2**}$ einen Methylrest, $R^{3**}$ die Gruppe —N($R^{4**}$)$R^{5**}$, $R^{4**}$ und $R^{5**}$ gemeinsam unter Einschluß des Stickstoffatoms eine in 4-Stellung durch eine Methylgruppe substituierte Piperazin-1-yl-gruppe und A** eine Methylengruppe bedeuten, sowie ihre pharmakologisch verträglichen Säureadditionssalze herstellt.

**Claims** (for the contracting States : BE, CH, DE, FR, GB, IT, LI, LU, NL, SE)

1. Substituted thienobenzodiazepinones of the general formula I

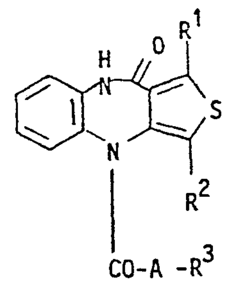

(I)

wherein

$R^1$ denotes a hydrogen atom or an alkyl radical with 1 to 4 carbon atoms,

$R^2$ represents a halogen atom or has one of the meanings of $R^1$,

$R^3$ denotes a halogen atom or the group $-N(R^4)R^5$,

$R^4$ denotes an alkyl radical with 1 to 4 carbon atoms or an alkenyl radical with 3 to 5 carbon atoms,

$R^5$ has one of the meanings of $R^4$ or represents the group $-(CH_2)_m-N(R^6)R^7$, or

$R^4$ and $R^5$ together, and with the inclusion of the nitrogen atom to which they are bonded, denote a morpholino group, a pyrrolidino group, a piperidino group, a hexahydroazepin-1-yl group, a piperazin-1-yl group which is optionally substituted in the 4-position by a methyl, ethyl or benzyl group, a 2,4-dimethyl-piperazin-1-yl group, or a hexahydro-1H-1,4-diazepin-1-yl group which is substituted in the 4-position by a methyl or ethyl group,

$R^6$ denotes an alkyl group with 1 to 4 carbon atoms,

$R^7$ denotes an alkyl group with 1 to 4 carbon atoms,

A denotes a straight-chain or branched alkylene group with 1 to 5 carbon atoms and

m denotes 2 or 3,

and their acid addition salts.

2. Substituted thienobenzodiazepinones according to Claim 1, characterised by the general formula I*

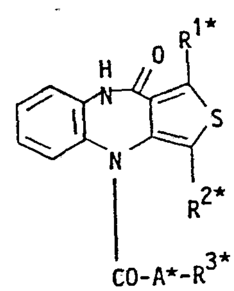

(I*)

wherein

$R^{1*}$ denotes a hydrogen atom or a methyl or ethyl radical,

$R^{2*}$ represents a chlorine atom or has one of the meanings of $R^{1*}$,

$R^{3*}$ denotes a chlorine atom and

A* denotes a straight-chain or branched alkylene group with 1 or 2 carbon atoms.

3. Substituted thienobenzodiazepinones according to Claim 1, characterised by the general formula I**

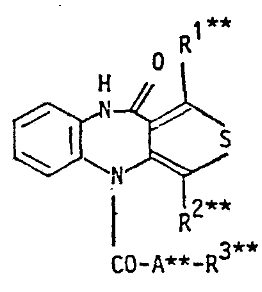

(I**)

wherein

$R^{1**}$ denotes a hydrogen atom or a methyl or ethyl radical,

$R^{2**}$ represents a chlorine atom or has one of the meanings of $R^{1**}$,
$R^{3**}$ denotes the group $—N(R^{4**})R^{5**}$,
$R^{4**}$ denotes an alkyl radical with 1 to 4 carbon atoms or an alkenyl radical with 3 to 4 carbon atoms,
$R^{5**}$ has the meaning of $R^{4**}$ or represents the group $—(CH_2)_{m**}—N(R^{6**})R^{7**}$, or
$R^{4**}$ and $R^{5**}$ together, with the inclusion of the nitrogen atom to which they are bonded, denote a morpholino group, a pyrrolidino group, a piperidino group, a hexahydroazepin-1-yl group, a piperazin-1-yl group which is optionally substituted in the 4-position by a methyl, ethyl or benzyl group, a 2,4-dimethyl-piperazin-1-yl group or a hexahydro-1H-1,4-diazepin-1-yl group which is substituted in the 4-position by a methyl or ethyl group,
$R^{6**}$ denotes a methyl or ethyl group,
$R^{7**}$ denotes a methyl or ethyl group,
m** denotes 2 or 3 and
A** denotes a straight-chain or branched alkylene group with 1 or 2 carbon atoms,
and their acid addition salts.

4. Compounds according to Claim 3, in which $R^{1**}$ denotes a hydrogen atom or a methyl or ethyl radical, $R^{2**}$ represents a chlorine atom or has one of the meanings of $R^{1**}$ ; $R^{3**}$ denotes the group $—N(R^{4**})R^{5**}$ ; $R^{4**}$ denotes a methyl or ethyl radical and $R^{5**}$ has the meaning of $R^{4**}$ or represents the group $—(CH_2)_{m**}—N(R^{6**})R^{7**}$, or $R^{4**}$ and $R^{5**}$ together, with the inclusion of the nitrogen atom, denote a pyrrolidino, piperidino or hexahydroazepin-1-yl radical, $R^{6**}$ and $R^{7**}$ denote a methyl or ethyl radical, m** denotes 2 and A** denotes a methylene group, and their pharmacologically acceptable acid addition salts.

5. Compounds according to Claim 3, in which $R^{1**}$ denotes a hydrogen atom or a methyl or ethyl radical, $R^{2**}$ represents a chlorine atom or has one of the meanings of $R^{1**}$ ; $R^{3**}$ denotes the group $—N(R^{4**})R^{5**}$, $R^{4**}$ and $R^{5**}$ together, with the inclusion of the nitrogen atom, denote a piperazin-1-yl group which is substituted in the 4-position by a methyl, ethyl or benzyl group, a 2,4-dimethylpiperazin-1-yl group, or a hexahydro-1H-1,4-diazepin-1-yl group which is substituted in the 4-position by a methyl or ethyl group ; and A** denotes a methylene group, and their pharmacologically acceptable acid addition salts.

6. Compounds according to Claim 3, in which $R^{1**}$ denotes a hydrogen atom or a methyl radical, $R^{2**}$ denotes a hydrogen atom or a methyl radical, $R^{3**}$ denotes the group $—N(R^{4**})R^{5**}$, $R^{4**}$ and $R^{5**}$ together, with inclusion of the nitrogen atom, denote a piperazin-1-yl group which is substituted in the 4-position by a methylene group and A** denotes a methylene group, and their pharmacologically acceptable acid addition salts.

7. Medicaments containing one or more thienobenzodiazepinones of the general formula Ia

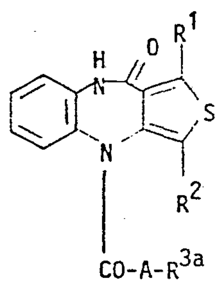

(Ia)

wherein
$R^1$ denotes a hydrogen atom or an alkyl radical with 1 to 4 carbon atoms,
$R^2$ represents a halogen atom or has one of the meanings of $R^1$,
$R^{3a}$ denotes the group $—N(R^4)R^5$,
$R^4$ denotes an alkyl radical with 1 to 4 carbon atoms or an alkenyl radical with 3 to 5 carbon atoms and
$R^5$ has one of the meanings of $R^4$ or represents the group $—(CH_2)_m—N(R^6)R^7$, or
$R^4$ and $R^5$ together, and with the inclusion of the nitrogen atom to which they are bonded, denote a morpholino group, a pyrrolidino group, a piperidino group, a hexahydroazepin-1-yl group, a piperazin-1-yl group which is optionally substituted in the 4-position by a methyl, ethyl or benzyl group, a 2,4-dimethyl-piperazin-1-yl group, or a hexahydro-1H-1,4-diazepin-1-yl group which is substituted in the 4-position by a methyl or ethyl group,
$R^6$ denotes an alkyl group with 1 to 4 carbon atoms,
$R^7$ denotes an alkyl group with 1 to 4 carbon atoms,
A denotes a straight-chain or branched alkylene group with 1 to 5 carbon atoms and
m denotes 2 or 3,
and/or their pharmacologically acceptable acid addition salts.

8. Process for the preparation of the substituted thienobenzodiazepinones of the general formula I

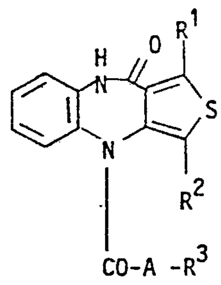

(I)

wherein

$R^1$ denotes a hydrogen atom or an alkyl radical with 1 to 4 carbon atoms,

$R^2$ represents a halogen atom or has one of the meanings of $R^1$,

$R^3$ denotes a halogen atom or the group —$N(R^4)R^5$,

$R^4$ denotes an alkyl radical with 1 to 4 carbon atoms or an alkenyl radical with 3 to 5 carbon atoms,

$R^5$ has one of the meanings of $R^4$ or represents the group —$(CH_2)_m$—$N(R^6)R^7$, or

$R^4$ and $R^5$ together, and with the inclusion of the nitrogen atom to which they are bonded, denote a morpholino group, a pyrrolidino group, a piperidino group, a hexahydroazepin-1-yl group, a piperazin-1-yl group which is optionally substituted in the 4-position by a methyl, ethyl or benzyl group, a 2,4-dimethyl-piperazin-1-yl group, or a hexahydro-1H-1,4-diazepin-1-yl group which is substituted in the 4-position by a methyl or ethyl group,

$R^6$ denotes an alkyl group with 1 to 4 carbon atoms,

$R^7$ denotes an alkyl group with 1 to 4 carbon atoms,

A denotes a straight-chain or branched alkylene group with 1 to 5 carbon atoms and

m denotes 2 or 3,

and their acid addition salts, characterised in that thienobenzodiazepinones of the general formula II

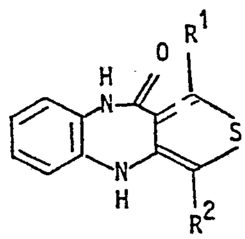

(II)

wherein $R^1$ and $R^2$ have the abovementioned meanings, are acylated and, if appropriate, are then aminated, and/or resulting bases are converted into the free base or into pharmacologically acceptable acid addition salts.

9. Process according to Claim 8, characterised in that the acylation is carried out with compounds of the general formulae Hal—A—CO—Hal (III) or (Hal—A—CO)$_2$O (IV), wherein Hal and Hal′ denote a halogen atom and A denotes an alkylene group with 1 to 5 carbon atoms.

10. Process according to Claim 8, characterised in that compounds of the general formula I wherein $R^3$ represents a halogen atom, and secondary amines of the general formula $HN(R^4)R^5$ (V), wherein $R^4$ and $R^5$ have the meaning given in Claim 8, are employed in the optional subsequent amination.

11. Process according to Claim 10, characterised in that piperazine or homopiperazine are employed as secondary amines V in the optional subsequent amination, and the obtained product of the general formula X

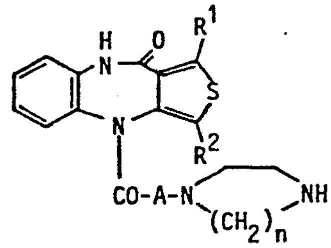

(X)

wherein $R^1$, $R^2$ and A have the meanings given in Claim 8 and n denotes 2 or 3, is methylated or ethylated.

12. Process according to Claim 8, characterised in that the acylation is carried out with compounds of the general formula Z—CO—A—$N(R^4)R^5$ (IX), wherein Z denotes a leaving group and A, $R^4$ and $R^5$ have the meanings given in Claim 8.

13. Substituted thienobenzodiazepinones of the general formula Ia, wherein the substituents have the meanings given in Claim 7, for use in the treatment and prophylaxis of illnesses based on disorders of the stomach or intestine.

14. Compound according to Claim 3, in which $R^{1**}$ denotes a hydrogen atom ; $R^{2**}$ denotes a methyl radical ; $R^{4**}$ and $R^{5**}$, and with the inclusion of the nitrogen atom, denote a piperazin-1-yl group which is substituted in the 4-position by a methyl group ; and A denotes a methylene group, and their pharmacologically acceptable acid addition salts.

**Claims** (for the Contracting State AT)

1. Process for the preparation of the substituted thieno-benzodiazepinones of the general formula I

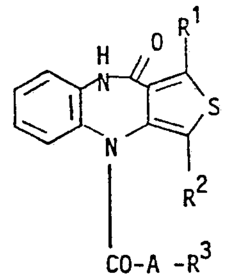

(I)

wherein

$R^1$ denotes a hydrogen atom or an alkyl radical with 1 to 4 carbon atoms,

$R^2$ represents a halogen atom or has one of the meanings of $R^1$,

$R^3$ denotes a halogen atom or the group $—N(R^4)R^5$,

$R^4$ denotes an alkyl radical with 1 to 4 carbon atoms or an alkenyl radical with 3 to 5 carbon atoms,

$R^5$ has one of the meanings of $R^4$ or represents the group $—(CH_2)_m—N(R^6)R^7$, or

$R^4$ and $R^5$ together, and with the inclusion of the nitrogen atom to which they are bonded, denote a morpholino group, a pyrrolidino group, a piperidino group, a hexahydroazepin-1-yl group, a piperazin-1-yl group which is optionally substituted in the 4-position by a methyl, ethyl or benzyl group, a 2,4-dimethyl-piperazin-1-yl group, or a hexahydro-1H-1,4-diazepin-1-yl group which is substituted in the 4-position by a methyl or ethyl group,

$R^6$ denotes an alkyl group with 1 to 4 carbon atoms,

$R^7$ denotes an alkyl group with 1 to 4 carbon atoms,

A denotes a straight-chain or branched alkylene group with 1 to 5 carbon atoms and

m denotes 2 or 3,

and their acid addition salts, characterised in that thienobenzodiazepinones of the general formula II

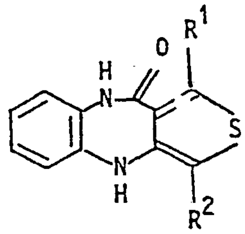

(II)

wherein $R^1$ and $R^2$ have the abovementioned meanings, are acylated and, if appropriate, are then aminated, and/or resulting bases are converted into the free base or into pharmacologically acceptable acid addition salts.

2. Process according to Claim 1, characterised in that the acylation is carried out with compounds of the general formulae Hal—A—CO—Hal′ (III) or $(Hal—A—CO)_2O$ (IV), wherein Hal and Hal′ denote a halogen atom and A denotes an alkylene group with 1 to 5 carbon atoms.

3. Process according to Claim 1, characterised in that compounds of the general formula I wherein $R^3$ represents a halogen atom, and secondary amines of the general formula $HN(R^4)R^5$ (V), wherein $R^4$ and $R^5$ have the meanings given in Claim 1, are employed in the optional subsequent amination.

4. Process according to Claim 3, characterised in that piperazine or homopiperazine are employed as secondary amines V in the optional subsequent amination, and the obtained product of the general formula X

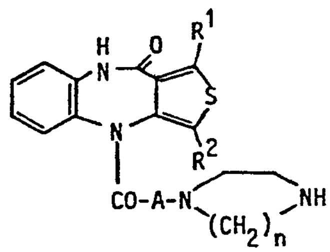

wherein $R^1$, $R^2$ and A have the meanings given in Claim 1 and n denotes 2 or 3, is methylated or ethylated.

5. Process according to Claim 1, characterised in that the acylation is carried out with compounds of the general formula Z—CO—A—N($R^4$)$R^5$ (IX), wherein Z denotes a leaving group and A, $R^4$ and $R^5$ have the meanings given in Claim 1.

6. Process according to Claims 1 and 2, characterised in that substituted thienobenzodiazepinones of the general formula I*

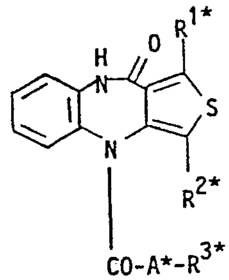

(I*)

wherein

$R^{1*}$ denotes a hydrogen atom, a methyl or ethyl radical,

$R^{2*}$ denotes a chlorine atom or has one of the meanings of $R^{1*}$,

$R^{3*}$ denotes a chlorine atom,

A* denotes a straight-chain or branched alkylene group with 1 or 2 carbon atoms, are prepared by reaction of thienobenzodiazepinones of the general formula II*

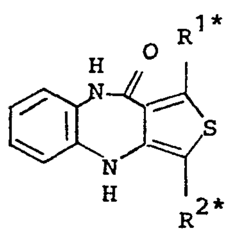

(II*)

wherein $R^{1*}$ and $R^{2*}$ have the abovementioned meanings, with acid derivatives III* Cl—A*—CO—Cl (III*) or IV* (Cl—A*—CO)$_2$O (IV*), wherein A* has the above mentioned meaning.

7. Process according to Claims 1 and 3, characterised in that substituted thienobenzodiazepinones of the general formula I**

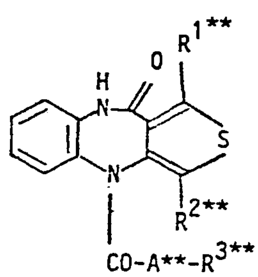

(I**)

wherein

$R^{1**}$ denotes a hydrogen atom, a methyl or ethyl radical,

$R^{2**}$ denotes a chlorine atom or has one of the meanings or $R^{1**}$,

$R^{3**}$ denotes the group —N($R^{4**}$)$R^{5**}$,

$R^{4**}$ denotes an alkyl radical with 1 to 4 carbon atoms or an alkenyl radical with 3 to 4 carbon atoms,

$R^{5**}$ has the meaning of $R^{4**}$ or represents the group —$(CH_2)_{m**}$—N($R^{6**}$)$R^{7**}$, or

$R^{4**}$ and $R^{5**}$ together, and with the inclusion of the nitrogen atom to which they are bonded,

denote a morpholino group, a pyrrolidino group, a piperidino group, a hexahydroazepin-1-yl group, a piperazin-1-yl group which is optionally substituted in the 4-position by a methyl, ethyl or benzyl group, 1 2,4-dimethyl-piperazin-1-yl group, a hexahydro-1H-1,4-diazepin-1-yl group which is substituted in the 4-position by a methyl or ethyl group,

$R^{6**}$ denotes a methyl or ethyl group,

$R^{7**}$ denotes a methyl or ethyl group,

m** denotes 2 or 3 and

A** denotes a straight-chain or branched alkylene group with 1 or 2 carbon atoms

and their acid addition salts are prepared by reaction of compounds of the general formula I**, wherein $R^{3**}$ denotes a chlorine atom, with amines of the general formula V** $HN(R^{4**})R^{5**}$ (V**), wherein $R^{4**}$ and $R^{5**}$ have the abovementioned meanings.

8. Process according to Claim 7, characterised in that compounds of the general formula I**, in which $R^{1**}$ denotes a hydrogen atom, a methyl or ethyl radical, $R^{2*}$ represents a chlorine atom or has one of the meanings of $R^{1**}$; $R^{3**}$ denotes the group $—N(R^{4**})R^{5**}$, $R^{4**}$ denotes a methyl or ethyl radical, $R^{5**}$ has the meaning of $R^{4**}$ or represents the group $—(CH_2)_{m**}—N(R^{6**})R^{7**}$, or $R^{4**}$ and $R^{5**}$ together, with the inclusion of the nitrogen atom, denote a pyrrolidino, piperidino or hexahydroazepin-1-yl radical, $R^{6**}$ and $R^{7**}$ denote a methyl or ethyl radical, m** denotes 2 and A** denotes a methylene group, and their parmacologically acceptable acid addition salts are prepared.

9. Process according to Claim 7, characterised in that compounds of the general formula I**, in which $R^{1**}$ denotes a hydrogen atom, a methyl or ethyl radical, $R^{2**}$ represents a chlorine atom or has one of the meanings of $R^{1**}$, $R^{3**}$ denotes the group $—N(R^{4**})R^{5**}$; $R^{4**}$ and $R^{5**}$ together, with the inclusion of the nitrogen atom, denote a piperazin-1-yl which is optionally substituted in the 4-position by a methyl, ethyl or benzyl group, a 2,4-dimethylpiperazin-1-yl group or a hexahydro-1H-1,4-diazepin-1-yl group which is substituted in the 4-position by a methyl or ethyl group and A** denotes a methylene group, and their pharmacologically acceptable acid addition salts are prepared.

10. Process according to Claim 7, characterised in that compounds of the general formula I**, in which $R^{1**}$ denotes a hydrogen atom or a methyl radical, $R^{2**}$ denotes a hydrogen atom or a methyl radical, $R^{3**}$ denotes the group $—N(R^{4**})R^{5**}$, $R^{4**}$ and $R^{5**}$ together, with inclusion of the nitrogen atom, denote a piperazin-1-yl group which is substituted in the 4-position by a methyl group and A** denotes a methylene group, and their pharmacologically acceptable acid addition salts are prepared.

11. Process according to Claims 1 and 4, characterised in that substituted thienobenzodiazepinones of the general formula I**

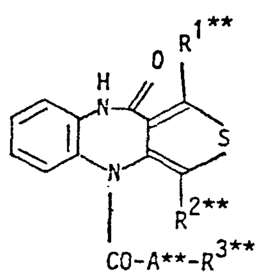

(I**)

wherein

$R^{1**}$ denotes a hydrogen atom, a methyl or ethyl radical,

$R^{2**}$ represents a chlorine atom or has one of the meanings of $R^{1**}$,

$R^{3**}$ denotes the group $—N(R^{4**})R^{5**}$,

$R^{4**}$ and $R^{5**}$ together, with the inclusion of the nitrogen atom to which they are bonded, denote a piperazin-1-yl group which is substituted in the 4-position by a methyl or ethyl group, or a hexahydro-1H-1,4-diazepin-1-yl group which is substituted in the 4-position by a methyl or ethyl group,

A** denotes a straight-chain or branched alkylene group with 1 or 2 carbon atoms,

and their addition salts are prepared by reaction of compounds of the general formula I**, wherein $R^{3**}$ denotes a chlorine atom, with piperazine or homopiperazine as amines of the general formula V** $HN(R^{4**})R^{5**}$ (V**) and methylation or ethylation of the obtained piperazinothienobenzodiazepinones of the general formula X**

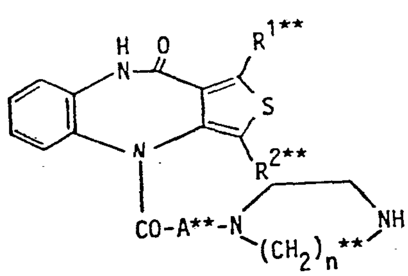

(X**)

wherein $R^{1**}$, $R^{2**}$ and A** have the abovementioned meanings and n** denotes 2 or 3.

12. Process according to Claim 11, characterised in that compounds of the general formula I**, wherein $R^{1**}$ denotes a hydrogen atom or a methyl radical, $R^{2**}$ has one of the meanings of $R^{1**}$, $R^{3**}$ denotes the group —N($R^{4**}$)$R^{5**}$, $R^{4**}$ and $R^{5**}$ together, with the inclusion of the nitrogen atom, denote a piperazin-1-yl group which is substituted in the 4-position by a methyl group, A** denotes a methylene group, and their pharmacologically acceptable acid addition salts are prepared.

13. Process according to Claims 1 and 5, characterised in that substituted thienobenzodiazepinones of the general formula I**

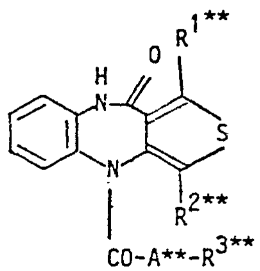

(I**)

wherein $R^{1**}$, $R^{2**}$, $R^{3**}$ and A** have the meanings given in Claim 7, are prepared by reaction of thienobenzodiazepinones of the general formula II**

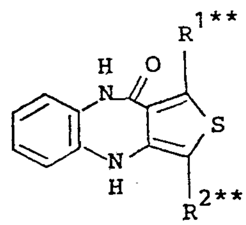

(II**)

wherein $R^{1**}$ and $R^{2**}$ have the abovementioned meanings, with reactive acid derivatives of the general formula IX** Z**—CO—A**—N($R^{4**}$)$R^{5**}$ (IX**), wherein Z** denotes a leaving group and A**, $R^{4**}$ and $R^{5**}$ have the meanings given in Claim 7.

14. Process according to Claim 7, characterised in that the compound of the general formula I**, wherein $R^{1**}$ denotes a hydrogen atom, $R^{2**}$ denotes a methyl radical, $R^{3**}$ denotes the group —N($R^{4**}$)$R^{5**}$, $R^{4**}$ and $R^{5**}$ together, with the inclusion of the nitrogen atom, denote a piperazin-1-yl group which is substituted in the 4-position by a methyl group, A** denotes a methylene group, and its pharmacologically acceptable acid addition salts are prepared.

15. Process according to Claim 11, characterised in that the compound of the general formula I**, wherein $R^{1**}$ denotes a hydrogen atom, $R^{2**}$ denotes a methyl radical, $R^{3**}$ denotes the group —N($R^{4**}$)$R^{5**}$, $R^{4**}$ and $R^{5**}$ together, with the inclusion of the nitrogen atom, denote a piperazin-1-yl group which is substituted in the 4-position by a methyl group, A** denotes a methylene group, and its pharmacologically acceptable acid addition salts are prepared.

16. Process according to Claim 13, characterised in that the compound of the general formula I*, wherein $R^{1**}$ denotes a hydrogen atom, $R^{2**}$ denotes a methyl radical, $R^{3**}$ denotes the group —N($R^{4**}R^{5**}$), $R^{4**}$ and $R^{5**}$ together, with the inclusion of the nitrogen atom, denote a piperazin-1-yl group which is substituted in the 4-position by a methyl group, A** denotes a methylene group, and its pharmacologically acceptable acid addition salts are prepared.

**Revendications** (pour les Etats contractants : BE, CH, DE, FR, GB, IT, LI, LU, NL, SE)

1. Thiénobenzodiazépinones substituées de la formule générale (I)

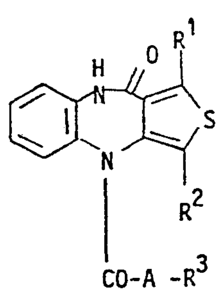

(I)

dans laquelle

$R^1$ représente un atome d'hydrogène ou un radical alkyle possédant de 1 à 4 atomes de carbone,

$R^2$ représente un atome d'halogène ou possède l'une des significations attribuées à $R^1$,

$R^3$ représente un atome d'halogène ou le radical —$N(R^4)R^5$ et

$R^4$ représente un radical alkyle possédant de 1 à 4 atomes de carbone ou un radical alcényle possédant de 3 à 5 atomes de carbone,

$R^5$ possède l'une des significations attribuées à $R^4$ ou représente un radical —$(CH_2)_m$—$N(R^6)R^7$, ou bien

$R^4$ et $R^5$ représentent en commun avec l'atome d'azote auquel ils sont attachés, un groupe morpholino, un groupe pyrrolidino, un groupe pipéridino, un groupe hexahydroazépin-1-yle, un groupe pipérazin-1-yle éventuellement substitué en position 4 par un radical méthyle, éthyle ou benzyle, un groupe 2,4-diméthylpipérazin-1-yle ou un groupe hexahydro-1H-1,4-diazépin-1-yle substitué en position 4 par un radical méthyle ou éthyle et

$R^6$ représente un groupe alkyle possédant de 1 à 4 atomes de carbone,

$R^7$ représente un groupe alkyle possédant de 1 à 4 atomes de carbone,

A représente un groupe alkylène à chaîne droite ou à chaîne ramifiée possédant de 1 à 5 atomes de carbone et

m est égal à 2 ou à 3,

comme aussi les sels d'addition d'acides de ces composés.

2. Thiénobenzodiazépinones substituées suivant la revendication 1, caractérisées en ce qu'ils répondent à la formule générale I*

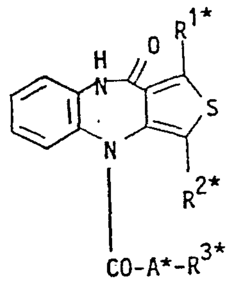

(I*)

dans laquelle

$R^{1*}$ représente un atome d'hydrogène, un radical méthyle ou éthyle,

$R^{2*}$ représente un atome de chlore ou possède l'une des significations attribuées à $R^{1*}$,

$R^{3*}$ représente un atome de chlore, et

A* représente un groupe alkylène à chaîne droite ou à chaîne ramifiée possédant 1 ou 2 atomes de carbone.

3. Thiénobenzodiazépinones substituées suivant la revendication 1, caractérisées en ce qu'ils répondent à la formule générale I**

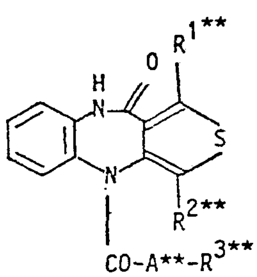

(I**)

dans laquelle

$R^{1**}$ représente un atome d'hydrogène, un radical méthyle ou éthyle,

$R^{2**}$ représente un atome de chlore ou possède l'une des significations attribuées à $R^{1**}$,

$R^{3**}$ représente le radical —$N(R^{4**})R^{5**}$ et

$R^{4**}$ représente un radical alkyle possédant de 1 à 4 atomes de carbone ou un radical alcényle possédant 3 ou 4 atomes de carbone,

$R^{5**}$ possède la signification attribuée à $R^{4**}$ ou représente un groupe —$(CH_2)_{m**}$—$N(R^{6**})R^{7**}$, ou bien

$R^{4**}$ et $R^{5**}$ représentent en commun avec l'atome d'azote auquel ils sont attachés, un groupe morpholino, un groupe pyrrolidino, un groupe pipéridino, un groupe hexahydroazépin-1-yle, un groupe pipérazin-1-yle éventuellement substitué en position 4 par un radical méthyle, éthyle ou benzyle, un groupe 2,4-diméthylpipérazin-1-yle ou un groupe hexahydro-1H-1,4-diazépin-1-yle substitué en position 4 par un radical méthyle ou éthyle et

$R^{6**}$ représente un groupe méthyle ou éthyle,

$R^{7**}$ représente un groupe méthyle ou éthyle,

m** est égal à 2 ou à 3,

A** représente un groupe alkylène à chaîne droite ou à chaîne ramifiée possédant 1 ou 2 atomes de carbone,

ainsi que les sels d'addition d'acides de ces composés.

4. Composés suivant la revendication 3, dans lesquels $R^{1**}$ représente un atome d'hydrogène, le radical méthyle ou éthyle, $R^{2**}$ représente un atome de chlore ou possède l'une des significations attribuées à $R^{1**}$, $R^{3**}$ représente le groupe $-N(R^{4**})R^{5**}$, $R^{4**}$ représente le radical méthyle ou éthyle, $R^{5**}$ possède la signification attribuée à $R^{4**}$ ou représente le groupe $(CH_2)_{m**}-N(R^{6**})R^{7**}$, ou bien $R^{4**}$ et $R^{5**}$ représentent, communément avec l'atome d'azote, un radical pyrrolidino, pipéridino ou hexahydroazépin-1-yle, $R^{6**}$ et $R^{7**}$ représente le radical méthyle ou éthyle, m** est égal à 2 et A** représente un radical méthylène, comme aussi les sels d'addition d'acides pharmacologiquement acceptables de ces composés.

5. Composés suivant la revendication 3, dans lesquels $R^{1**}$ représente un atome d'hydrogène, le radical méthyle ou éthyle, $R^{2**}$ représente un atome de chlore ou possède l'une des significations attribuées à $R^{1**}$, $R^{3**}$ représente le groupe $-N(R^{4**})R^{5**}$, $R^{4**}$ et $R^{5**}$ représentent, communément avec l'atome d'azote, un radical pipérazin-1-yle substitué en position 4 par un radical méthyle, éthyle ou benzyle, un radical 2,4-diméthylpipérazin-1-yle ou un radical hexahydro-1H-1,4-diazépin-1-yle substitué en position 4 par un radical méthyle ou éthyle et A** représente un radical méthylène, comme aussi les sels d'addition d'acides pharmacologiquement acceptables de ces composés.

6. Composés suivant la revendication 3, dans lesquels $R^{1**}$ représente un atome d'hydrogène ou le radical méthyle, $R^{2**}$ représente un atome d'hydrogène ou un radical méthyle, $R^{3**}$ représente le radical $-N(R^{4**})R^{5**}$, $R^{4**}$ et $R^{5**}$ représentent, communément avec l'atome d'azote, un groupe pipérazin-1-yle substitué en position 4 par un radical méthyle et A** représente un groupe méthylène, comme aussi les sels d'addition d'acides pharmacologiquement acceptables de ces composés.

7. Médicaments qui contiennent une ou plusieurs thiénobenzodiazépinones de la formule générale Ia

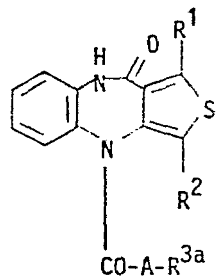

(Ia)

dans laquelle

$R^1$ représente un atome d'hydrogène ou un radical alkyle possédant de 1 à 4 atomes de carbone,

$R^2$ représente un atome d'halogène ou possède l'une des significations attribuées à $R^1$,

$R^{3a}$ représente le groupe $-N(R^4)R^5$ et

$R^4$ représente un radical alkyle possédant de 1 à 4 atomes de carbone ou un reste alcényle possédant de 3 à 5 atomes de carbone,

$R^5$ possède l'une des significations attribuées à $R^4$ ou représente le radical $-(CH_2)_m-N(R^6)R^7$, ou bien

$R^4$ et $R^5$ représentent en commun avec l'atome d'azote auquel ils sont attachés, un groupe morpholino, un groupe pyrrolidino, un groupe pipéridino, un groupe hexahydro-azépin-1-yle, un groupe pipérazin-1-yle éventuellement substitué en position 4 par un radical méthyle, éthyle, ou benzyle, un groupe 2,4-diméthyl-pipérazin-1-yle, ou un groupe hexahydro-1H-1,4-diazépin-1-yle substitué en position 4 par un groupe méthyle ou éthyle et

$R^6$ représente un radical alkyle possédant de 1 à 4 atomes de carbone,

$R^7$ représente un radical alkyle possédant de 1 à 4 atomes de carbone,

A représente un radical alkylène à chaîne droite ou à chaîne ramifiée possédant de 1 à 5 atomes de carbone et

m est égal à 2 ou à 3,

et/ou leurs sels d'addition d'acides pharmacologiquement acceptables.

8. Procédé de préparation de thiénobenzodiazépinones substituées de la formule générale (I)

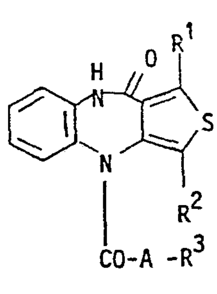

(I)

dans laquelle

$R^1$ représente un atome d'hydrogène ou un radical alkyle possédant de 1 à 4 atomes de carbone,

$R^2$ représente un atome d'halogène ou possède l'une des significations attribuées à $R^1$,

$R^3$ représente un atome d'halogène ou le radical $—N(R^4)R^5$ et

$R^4$ représente un radical alkyle possédant de 1 à 4 atomes de carbone ou un radical alcényle possédant de 3 à 5 atomes de carbone,

$R^5$ possède l'une des significations attribuées à $R^4$ ou représente un radical $—(CH_2)_m—N(R^6)R^7$, ou bien

$R^4$ et $R^5$ représentent en commun avec l'atome d'azote auquel ils sont attachés, un groupe morpholino, un groupe pyrrolidino, un groupe pipéridino, un groupe hexahydroazépin-1-yle, un groupe pipérazin-1-yle éventuellement substitué en position 4 par un radical méthyle, éthyle ou benzyle, un groupe 2,4-diméthylpipérazin-1-yle ou un groupe hexahydro-1H-1,4-diazépin-1-yle substitué en position 4 par un radical méthyle ou éthyle et

$R^6$ représente un groupe alkyle possédant de 1 à 4 atomes de carbone,

$R^7$ représente un groupe alkyle possédant de 1 à 4 atomes de carbone,

A représente un groupe alkylène à chaîne droite ou à chaîne ramifiée possédant de 1 à 5 atomes de carbone et

m est égal à 2 ou à 3,

comme aussi de leurs sels d'addition d'acides, caractérisé en ce que l'on procède à l'acylation de thiénobenzodiazépinones de la formule générale II

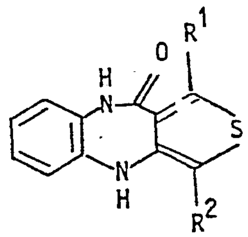

(II)

dans laquelle $R^1$ et $R^2$ possèdent les significations qui leur ont été attribuées plus haut et en ce qu'éventuellement l'on soumet les produits ainsi obtenus à une amination et/ou en ce que l'on convertit les bases obtenues en les sels d'addition d'acides ou en ce que l'on transforme les sels d'addition d'acides obtenus en les bases libres ou en les sels d'addition d'acides pharmacologiquement acceptables.

9. Procédé suivant la revendication 8, caractérisé en ce que l'on réalise l'acylation avec des composés de la formule générale Hal—A—CO—Hal' (III) ou $(Hal—A—CO)_2O$ (IV) dans laquelle Hal et Hal' représentent des atomes d'halogène et A représente un groupe alkylène possédant de 1 à 5 atomes de carbone.

10. Procédé suivant la revendication 8, caractérisé en ce qu'au cours de l'éventuelle amination subséquente, on utilise des composés de la formule générale I dans laquelle $R^3$ représente un atome d'halogène et des amines secondaires de la formule générale $HN(R^4)R^5$ (V), dans laquelle $R^4$ et $R^5$ possèdent les significations qui leur ont été attribuées dans la revendication 8.

11. Procédé suivant la revendication 10, caractérisé en ce qu'au cours de l'éventuelle amination subséquente, on utilise de la pipérazine ou de l'homopipérazine à titre d'amines secondaires V et en ce que l'on méthyle ou éthyle le produit de réaction obtenu de la formule générale X

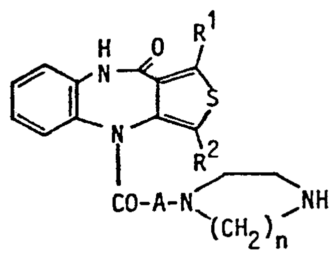

(X)

dans laquelle $R^1$, $R^2$ et A possèdent les significations qui leur ont été attribuées dans la revendication 8, et n est égal à 2 ou à 3.

12. Procédé suivant la revendication 8, caractérisé en ce que l'on réalise l'acylation avec des composés de la formule générale $Z—CO—A—N(R^4)R^5$ (IX), dans laquelle A, $R^4$ et $R^5$ possèdent les significations qui leur ont été attribuées dans la revendication 8 et Z représente un groupe scindable.

13. Thiénobenzodiazépinones substituées de la formule générale Ia dans laquelle les substituants possèdent les significations qui leur ont été attribuées dans la revendication 7, pour la mise en œuvre du traitement et de la prophylaxie de maladies dues à des affections de l'estomac ou de l'intestin.

14. Composé suivant la revendication 3, dans lequel $R^{1**}$ représente un atome d'hydrogène, $R^{2**}$ représente le radical méthyle, $R^{4**}$ et $R^{5**}$ représentent, communément avec l'atome d'azote, un groupe pipérazin-1-yle substitué en position 4 par un radical méthyle et A** représente un radical méthylène, comme aussi les sels d'addition d'acides pharmacologiquement acceptables de ce composé.

**Revendications** (pour l'Etat contractant AT)

1. Procédé de préparation de thiénobenzodiazépinones substituées de la formule générale (I)

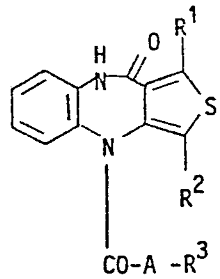

(I)

dans laquelle

$R^1$ représente un atome d'hydrogène ou un radical alkyle possédant de 1 à 4 atomes de carbone,
$R^2$ représente un atome d'halogène ou possède l'une des significations attribuées à $R^1$,
$R^3$ représente un atome d'halogène ou le radical $—N(R^4)R^5$ et
$R^4$ représente un radical alkyle possédant de 1 à 4 atomes de carbone ou un radical alcényle possédant de 3 à 5 atomes de carbone,
$R^5$ possède l'une des significations attribuées à $R^4$ ou représente un radical $—(CH_2)_m—N(R^6)R^7$, ou bien
$R^4$ et $R^5$ représentent en commun avec l'atome d'azote auquel ils sont attachés, un groupe morpholino, un groupe pyrrolidino, un groupe pipéridino, un groupe hexahydroazépin-1-yle, un groupe pipérazin-1-yle éventuellement substitué en position 4 par un radical méthyle, éthyle ou benzyle, un groupe 2,4-diméthylpipérazin-1-yle ou un groupe hexahydro-1H-1,4-diazépin-1-yle substitué en position 4 par un radical méthyle ou éthyle et
$R^6$ représente un groupe alkyle possédant de 1 à 4 atomes de carbone,
$R^7$ représente un groupe alkyle possédant de 1 à 4 atomes de carbone,
A représente un groupe alkylène à chaîne droite ou à chaîne ramifiée possédant de 1 à 5 atomes de carbone et
m est égal à 2 ou à 3,

comme aussi de leurs sels d'addition d'acides, caractérisé en ce que l'on procède à l'acylation de thiénobenzodiazépinones de la formule générale II

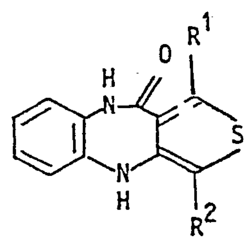

(II)

dans laquelle $R^1$ et $R^2$ possèdent les significations qui leur ont été attribuées plus haut et en ce qu'éventuellement l'on soumet les produits ainsi obtenus à une amination et/ou en ce que l'on convertit les bases obtenues en les sels d'addition d'acides ou en ce que l'on transforme les sels d'addition d'acides obtenus en les bases libres ou en les sels d'addition d'acides pharmacologiquement acceptables.

2. Procédé suivant la revendication 1, caractérisé en ce que l'on réalise l'acylation avec des composés de la formule générale Hal—A—CO—Hal' (III) ou $(Hal—A—CO)_2O$ (IV) dans laquelle Hal et Hal' représentent des atomes d'halogène et A représente un groupe alkylène possédant de 1 à 5 atomes de carbone.

3. Procédé suivant la revendication 1, caractérisé en ce qu'au cours de l'éventuelle amination subséquente, on utilise des composés de la formule générale I dans laquelle $R^3$ représente un atome d'halogène et des amines secondaires de la formule générale $HN(R^4)R^5$ (V), dans laquelle $R^4$ et $R^5$ possèdent les significations qui leur ont été attribuées dans la revendication 1.

4. Procédé suivant la revendication 3, caractérisé en ce qu'au cours de l'éventuelle amination subséquente, on utilise de la pipérazine ou de l'homopipérazine à titre d'amines secondaires V et en ce que l'on méthyle ou éthyle le produit de réaction obtenu de la formule générale X

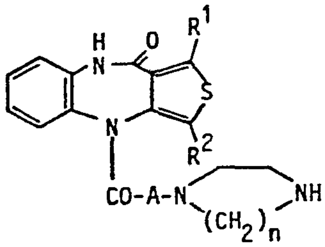

(X)

dans laquelle $R^1$, $R^2$ et A possèdent les significations qui leur ont été attribuées dans la revendication 1, et n est égal à 2 ou à 3.

5. Procédé suivant la revendication 1, caractérisé en ce que l'on réalise l'acylation avec des composés de la formule générale Z—CO—A—N($R^4$)$R^5$ (IX), dans laquelle A, $R^4$ et $R^5$ possèdent les significations qui leur ont été attribuées dans la revendication 1 et Z représente un groupe scindable.

6. Procédé suivant les revendications 1 et 2, caractérisé en ce que l'on prépare des thiénobenzodiazépinones substituées de la formule générale I*

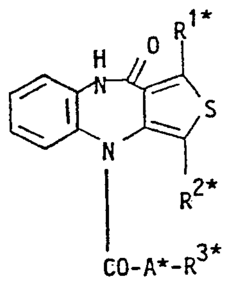

(I*)

dans laquelle

$R^{1*}$ représente un atome d'hydrogène, un radical méthyle ou éthyle,

$R^{2*}$ représente un atome de chlore ou possède l'une des significations attribuées à $R^{1*}$,

$R^{3*}$ représente un atome de chlore, et

A* représente un groupe alkylène à chaîne droite ou à chaîne ramifiée possédant 1 ou 2 atomes de carbone,

par la réaction de thiénobenzodiazépinones de la formule générale II*

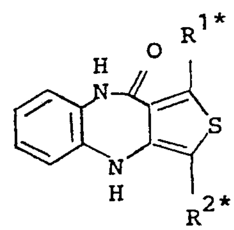

(II*)

dans laquelle $R^{1*}$ et $R^{2*}$ possèdent les significations qui leur ont été précédemment attribuées, sur des dérivés d'acides III* Cl—A*—CO—Cl (III*) ou IV* (Cl—A*—CO)$_2$O (IV*) dans laquelle A* possède les significations qui lui ont été précédemment attribuées.

7. Procédé suivant les revendications 1 et 3, caractérisé en ce que l'on prépare des thiénobenzodiazépinones substituées de la formule générale I**

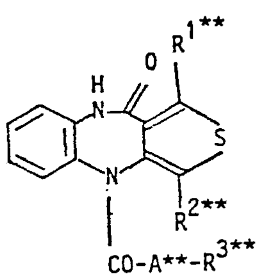

(I**)

dans laquelle

$R^{1**}$ représente un atome d'hydrogène, un radical méthyle ou éthyle,

$R^{2**}$ représente un atome de chlore ou possède l'une des significations attribuées à $R^{1**}$,

$R^{3**}$ représente le radical $—N(R^{4**})R^{5**}$ et

$R^{4**}$ représente un radical alkyle possédant de 1 à 4 atomes de carbone ou un radical alcényle possédant 3 ou 4 atomes de carbone,

$R^{5**}$ possède la signification attribuée à $R^{4**}$ ou représente un groupe $—(CH_2)_{m**}—N(R^{6**})R^{7**}$, ou bien

$R^{4**}$ et $R^{5**}$ représentent en commun avec l'atome d'azote auquel ils sont attachés, un groupe morpholino, un groupe pyrrolidino, un groupe pipéridino, un groupe hexahydroazépin-1-yle, un groupe pipérazin-1-yle éventuellement substitué en position 4 par un radical méthyle, éthyle ou benzyle, un groupe 2,4-diméthyl-pipérazin-1-yle ou un groupe hexahydro-1H-1,4-diazépin-1-yle substitué en position 4 par un radical méthyle ou éthyle et

$R^{6**}$ représente un groupe méthyle ou éthyle,

$R^{7**}$ représente un groupe méthyle ou éthyle,

m** est égal à 2 ou à 3,

A** représente un groupe alkylène à chaîne droite ou à chaîne ramifiée possédant 1 ou 2 atomes de carbone,

et de leurs sels d'addition d'acides, par la réaction des composés de la formule générale I** dans laquelle $R^{3**}$ représente un atome de chlore, sur des amines de la formule générale V** $HN(R^{4**})R^{5**}$ (V**), dans laquelle $R^{4**}$ et $R^{5**}$ possèdent les significations qui leur ont été précédemment attribuées.

8. Procédé suivant la revendication 7, caractérisé en ce que l'on prépare des composés de la formule générale I** dans laquelle $R^{1**}$ représente un atome d'hydrogène, le radical méthyle ou éthyle, $R^{2**}$ représente un atome de chlore ou possède l'une des significations attribuées à $R^{1**}$, $R^3$ représente le groupe $—N(R^{4**})R^{5**}$, $R^{4**}$ représente le radical méthyle ou éthyle, $R^{5**}$ possède la signification attribuée à $R^{4**}$ ou représente le groupe $—(CH_2)_{m**}—N(R^{6**})R^{7**}$, ou bien $R^{4**}$ et $R^{5**}$ représentent, communément avec l'atome d'azote, un radical pyrrolidino, pipéridino ou hexahydroazépin-1-yle, $R^{6**}$ et $R^{7**}$ représentent le radical méthyle ou éthyle, m** est égal à 2 et A** représente un groupe méthylène, comme aussi les sels d'addition d'acides pharmacologiquement acceptables de ces composés.

9. Procédé suivant la revendication 7, caractérisé en ce que l'on prépare des composés de la formule générale I** dans laquelle $R^{1**}$ représente un atome d'hydrogène ou le radical méthyle ou éthyle, $R^{2**}$ représente un atome de chlore ou possède l'une des significations attribuées à $R^{1**}$, $R^{3**}$ représente le groupe $—N(R^{4**})R^{5**}$, $R^{4**}$ et $R^{5**}$, représentent, communément avec l'atome d'azote, un radical pipérazin-1-yle substitué en position 4 par un radical méthyle, éthyle, ou benzyle, un groupe 2,4-diméthyl-pipérazin-1-yle ou un groupe hexahydro-1H-1,4-diazépin-1-yle substitué en position 4 par un radical méthyle ou éthyle et A** représente un groupe méthylène, comme aussi les sels d'addition d'acides pharmacologiquement acceptables de ces composés.

10. Procédé suivant la revendication 7, caractérisé en ce que l'on prépare des composés de la formule générale I** dans laquelle $R^{1**}$ représente un atome d'hydrogène ou le radical méthyle, $R^{2**}$ représente un atome d'hydrogène ou le radical méthyle, $R^{3**}$ représente le groupe $—N(R^{4**})R^{5**}$, $R^{4**}$ et $R^{5**}$ représentent, communément avec l'atome d'azote, un radical pipérazin-1-yle substitué en position 4 par un radical méthyle et A** représente un radical méthylène, comme aussi les sels d'addition pharmacologiquement acceptables de ces composés.

11. Procédé suivant les revendications 1 et 4, caractérisé en ce que l'on prépare des thiénobenzodiazépinones substituées de la formule générale I**

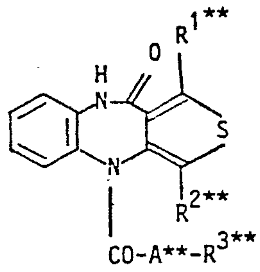

(I**)

dans laquelle

$R^{1**}$ représente un atome d'hydrogène, le radical méthyle ou éthyle,

$R^{2**}$ représente un atome de chlore ou possède l'une des significations attribuées à $R^{1**}$,

$R^{3**}$ représente le groupe $—N(R^{4**})R^{5**}$,

$R^{4**}$ et $R^{5**}$ représentent, communément avec l'atome d'azote auquel ils sont attachés, un groupe pipérazin-1-yle substitué par un radical méthyle ou éthyle en position 4, ou un groupe hexahydro-1H-1,4-diazépin-1-yle substitué par un groupe méthyle ou éthyle en position 4,

A** représente un groupe alkylène à chaîne droite ou à chaîne ramifiée possédant 1 ou 2 atomes de carbone et leurs sels d'addition d'acides, par la réaction de composés de la formule générale I** dans laquelle $R^{3**}$ représente un atome de chlore, sur de la pipérazine ou de l'homopipérazine à titre d'amine de la formule générale V** et la méthylation ou l'éthylation des pipérazinothiénobenzodiazépinones obtenues de la formule générale X**

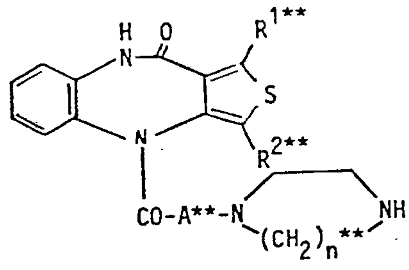

(X**)

dans laquelle $R^{1**}$, $R^{2**}$ et A** possèdent les significations qui leur ont été précédemment attribuées et n** est égal à 2 ou à 3.

12. Procédé suivant la revendication 11, caractérisé en ce que l'on prépare des composés de la formule générale I** dans laquelle $R^{1**}$ représente un atome d'hydrogène ou le radical méthyle, $R^{2**}$ possède l'une des significations attribuées à $R^{1**}$, $R^{3**}$ représente le groupe —N($R^{4**}$)$R^{5**}$, $R^{4**}$ et $R^{5**}$ représentent, communément avec l'atome d'azote, un groupe pipérazin-1-yle substitué par un groupe méthyle en position 4 et A** représente un groupe méthylène, comme aussi les sels d'addition d'acides pharmacologiquement acceptables de ces composés.

13. Procédé suivant les revendications 1 et 5, caractérisé en ce que l'on prépare des thiénobenzodiazépinones substituées de la formule générale I**

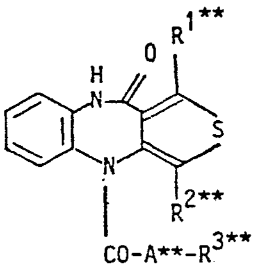

(I**)

dans laquelle $R^{1**}$, $R^{2**}$, $R^{3**}$ et A** possèdent les significations qui leur ont été attribuées dans la revendication 7, par la réaction de thiénobenzodiazépinones de la formule générale II**

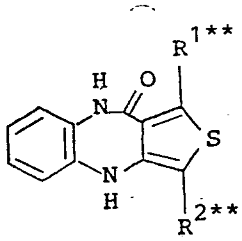

(II**)

dans laquelle $R^{1**}$ et $R^{2**}$ possèdent les significations qui leur ont été précédemment attribuées, sur des dérivés d'acides réactifs de la formule générale IX** Z**—CO—A**—N—($R^{4**}$)$R^{5**}$ (IX**), dans laquelle Z** représente un groupe scindable et A**, $R^{4**}$ et $R^{5**}$ possèdent les significations qui leur ont été attribuées dans la revendication 7.

14. Procédé suivant la revendication 7, caractérisé en ce que l'on prépare le composé de la formule générale I** dans laquelle $R^{1**}$ représente un atome d'hydrogène, $R^{2**}$ représente le radical méthyle, $R^{3**}$ représente le groupe —N($R^{4**}$)$R^{5**}$, $R^{4**}$ et $R^{5**}$ représentent, communément avec l'atome d'azote, un radical pipérazin-1-yle substitué par un groupe méthyle en position 4 et A** représente un groupe méthylène, comme aussi les sels d'addition d'acides pharmacologiquement acceptables de ces composés.

15. Procédé suivant la revendication 11, caractérisé en ce que l'on prépare le composé de la formule générale I** dans laquelle $R^{1**}$ représente un atome d'hydrogène, $R^{2**}$ représente le radical méthyle, $R^{3**}$ représente le groupe —N($R^{4**}$)$R^{5**}$, $R^{4**}$ et $R^{5**}$ représentent, communément avec l'atome d'azote, un radical pipérazin-1-yle substitué par un groupe méthyle en position 4 et A** représente un groupe méthylène, comme aussi les sels d'addition d'acides pharmacologiquement acceptables de ces composés.

16. Procédé suivant la revendication 13, caractérisé en ce que l'on prépare le composé de la formule générale I** dans laquelle $R^{1**}$ représente un atome d'hydrogène, $R^{2**}$ représente le radical méthyle, $R^{3**}$ représente le groupe —N($R^{4**}$)$R^{5**}$, $R^{4**}$ et $R^{5**}$ représentent, communément avec l'atome d'azote, un radical pipérazin-1-yle substitué par un radical méthyle en position 4 et A** représente un radical méthylène, comme aussi les sels d'addition d'acides pharmacologiquement acceptables de ces composés.